# EUROPÄISCHE PATENTANMELDUNG

(11) **EP 1 916 254 A2**
(43) Veröffentlichungstag der Anmeldung: **30.04.2008**
(21) Anmeldenummer: 08075102.7
(22) Anmeldetag: 27.11.2002

(54) **17alpha-Alkyl-17beta-oxy-estratriene und Zwischenprodukte zu deren Herstellung, Verwendung der 17alpha-Alkyl-17beta-oxy-estratriene zur Herstellung von Arzneimitteln sowie pharmazeutische Präparate**

(30) Priorität: 27.11.2001 DE 10159217
(62) Teilanmeldung aus: 02795080.7
(71) Anmelder: Bayer Schering Pharma Aktiengesellschaft, 13353 Berlin (DE)
(72) Erfinder: Bohlmann, Rolf, 14055 Berlin (DE); Heinrich, Nikolaus, 12159 Berlin (DE); Jautelat, Rolf, 16548 Glienicke-Nordbahn (DE); Kroll, Jörg, 12157 Berlin (DE); Petrov, Orlin, 14195 Berlin (DE); Reichel, Andreas, 10179 Berlin (DE); Hoffmann, Jens, 16567 Mühlenbeck (DE); Lichtner, Rosemarie, 10823 Berlin (DE)

(57) **Zusammenfassung**

Die Erfindung betrifft antiestrogen wirksame 17α-Alkyl-17β-oxy-estra-1,3,5(10)-triene mit der allgemeinen Formel I. Ausserdem betrifft die Erfindung auch 17-Oxo-estra-1,3,5(10)-triene sowie 17β-Hydroxy-estra-1,3,5(10)-triene als Zwischenprodukte bei der Herstellung der erfindungsgemässen Estratriene. Ferner betrifft die Erfindung die Verwendung der 17α-Alkyl-17β-oxy-estratriene zur Herstellung von Arzneimitteln sowie pharmazeutische Präparate, enthaltend mindestens ein 17α-Alkyl-17β-oxy-estratrien sowie mindestens einen pharmazeutisch verträglichen Träger.

## Beschreibung

Die Erfindung betrifft 17α-Alkyl-17β-oxy-estratriene und Zwischenprodukte zu deren Herstellung, Verwendung der 17α-Alkyl-17β-oxy-estratriene zur Herstellung von Arzneimitteln sowie diese Verbindungen enthaltende pharmazeutische Präparate.

Die erfindungsgemässen Verbindungen weisen antiestrogene Wirksamkeit auf, d.h. diese Stoffe üben Hemmwirkungen gegenüber Estrogenen aus. Derartige Stoffe sind bereits zahlreich beschrieben worden.

Beispielsweise sind antiestrogen-wirkende Verbindungen aus EP 0 138 504 B1 bekannt. Es handelt sich danach im wesentlichen um Estra-1,3,5(10)-trien-Derivate, die in 3-Stellung u.a. mit Hydroxy oder Alkoxy, in 17β-Stellung mit Hydroxy und in 17α-Stellung u.a. mit Wasserstoff oder Alkyl substituiert sind. Diese Verbindungen weisen in 7α-Stellung ferner eine Alkyl-Seitenkette auf, die teilweise fluoriert sein kann und die u.a. durch Amido-, Amino-, Amin-N-oxid-, Oxy-, Sulfanyl-, Sulfinyl- und/oder Sulfonylgruppen unterbrochen sein kann.

In WO 99/33855 A1 sind 11β-Halogen-7α-substituierte Estra-1,3,5(10)-triene beschrieben. die in 3- und in 17-Stellung Hydroxygruppen aufweisen können. Die 7α-Seitenkette ist eine teilweise fluorierte, gegebenenfalls ungesättigte Kohlenwasserstoffkette, die durch ein AminStickstoffatom oder eine Sulfanyl-, Sulfinyl- oder Sulfonylgruppe unterbrochen ist.

Weitere Verbindungen sind in WO 98/07740 A1 beschrieben. Es handelt sich hierbei um substituierte 7α-(ξ-Aminoalkyl)-estra-1,3,5(10)-triene. Diese Verbindungen weisen in 3-Stellung vorzugsweise eine Hydroxy-, Methoxy- oder Acetyloxygruppe und in 17α- und/oder 17β-Stellung vorzugsweise eine Methyl- bzw. Trifluormethylgruppe auf. In 11β-Stellung ist vorzugsweise ein Fluoratom vorgesehen und in 7α-Stellung eine Alkyl-Seitenkette, die endständig zumindest teilweise fluoriert ist und durch ein Amin-N-Atom und durch eine Sulfanyl-, Sulfinyl- oder Sulfonylgruppe unterbrochen ist.

In WO 97/45441 A1 sind 7α-(5-Methylaminopentyl)-estra-1,3,5(10)-triene offenbart, die in 3-Stellung und in 17β-Stellung eine Hydroxygruppe aufweisen. In 17α-Stellung kann eine Methyl- oder Ethinylgruppe vorgesehen sein. Ferner kann das Estratriengrundgerüst auch in 2-Stellung mit einem Fluoratom substituiert sein.

Es hat sich herausgestellt, dass die bekannten Verbindungen bei der Applikation eine Vielfalt biologisch sehr aktiver Metaboliten bilden. Die Bildung dieser Metaboliten führt zu unerwünschten Wirkungen und damit zu einem unkontrollierbaren Wirkungsspektrum. Insbesondere können sich Nebenwirkungen einstellen, oder die gewünschte Primärwirkung (antiestrogene Wirkung) wird durch spontane Bildung dieser Metaboliten unkontrollierbar. Ausserdem ist die Verträglichkeit der bekannten Verbindungen bei oraler Applikation nicht befriedigend. Es hat sich insbesondere herausgestellt, dass die bekannten Verbindungen die Anreicherung von alveolaren Makrophagen begünstigen.

Der vorliegenden Erfindung liegt von daher die Aufgabe zugrunde, antiestrogene Verbindungen zu finden, deren Metabolismus kontrollierbar ist und die daher keine oder nur wenige biologisch aktive Metaboliten bilden. Ausserdem ist es erwünscht, dass die Verträglichkeit der gesuchten Verbindungen bei oraler Gabe befriedigend ist und bei deren Darreichung u.a. sich alveolare Makrophagen nicht oder zumindest nur in geringem Umfange anreichern.

Diese Aufgabe wird gelöst durch neuartige 17α-Alkyl-17β-oxy-estratriene nach Anspruch 1, ferner durch neuartige 17β-Oxy-estratriene nach Anspruch 18 sowie 17-Oxo-Estratriene nach Anspruch 20, die jeweils als Zwischenprodukte zur Herstellung der erfindungsgemässen 17α-Alkyl-17β-oxy-estratriene eingesetzt werden können, ferner durch die Verwendung der 17α-Alkyl-17β-oxy-estratriene zur Herstellung von Arzneimitteln nach Anspruch 16 und durch die erfindungsgemässen Verbindungen enthaltende pharmazeutische Zusammensetzungen nach Anspruch 17.

Die erfindungsgemässen 17α-Alkyl-17β-oxy-estratriene haben die allgemeine Formel I:

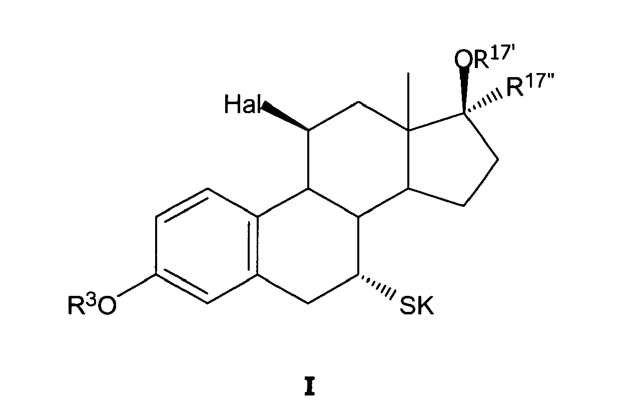

Darin bedeuten im einzelnen:
**Hal** = F oder Cl; dieser Rest ist in 11 β-Position an das Estratriengrundgerüst gebunden;
**R³** = Wasserstoff, C₁- - C₄-Alkyl, -Alkanoyl oder cyclischer C₃- - C₇-Ether mit einem O-Atom,
**R^{17'}** = Wasserstoff, C₁- - C₄-Alkyl und C₁- - C₄-Alkanoyl
**R^{17"}** = C₁- - C₄-Alkyl, C₁ - C₄-Alkinyl sowie ein zumindest teilweise fluorierter Alkylrest, wobei **R^{17'}**-O in 17β-Position und **R^{17"}** in 17α-Position an das Estratriengrundgerüst gebunden ist;
**SK = U-V-W-X-Y-Z-E,** wobei diese Gruppierung über **U** in 7α-Position an das Estratriengerüst gebunden ist.

In der Seitenkette haben die Symbole **U, V, W, X, Y, Z** und **E** die folgenden Bedeutungen:
**U** stellt entweder einen gerad- oder verzweigtkettigen C₁- - C₁₃-Alkylen-, -Alkenylen- oder -Alkinylenrest oder die Gruppe **A-B** dar,
   wobei A an das Estratriengrundgerüst gebunden ist und einen über -CH₂- an das Estratriengrundgerüst gebundenen Benzylidenrest, einen Phenylenrest oder einen über die Alkylgruppe an das Estratriengrundgerüst gebundenen C₁- - C₃-Alkylarylrest darstellt und
   **B** für einen gerad- oder verzweigtkettigen C₁- - C₁₃-Alkylen-, -Alkenylen- oder -Alkinylenrest steht und
   wobei **A** und **B** auch über ein O-Atom miteinander verbunden sein können.
**V** stellt eine CH₂- oder eine C(O)-Gruppe dar.
**W** ist eine N⁺(O-)(**R⁶**)-Gruppe oder ein Azolidinylen-N-Oxid-Ring,
   wobei der Azolidinylen-N-Oxid-Ring mindestens ein C-Atom der Gruppierung X einschliesst,
   wobei ferner **R⁶** entweder H oder CH₂-**R⁷** oder C(O)-**R⁷** ist, worin **R⁷** folgendes bedeuten kann:
   a) Wasserstoff oder
   b) einen gerad- oder verzweigtkettigen, nichtfluorierten oder zumindest teilweise fluorierten C₁- - C₁₄-Alkyl-, -Alkenyl- oder -Alkinylrest, der einfach oder mehrfach hydroxyliert sein kann und der durch ein bis drei der Heteroatome -O- und -S- und/oder der Gruppierungen -**NR⁹**- unterbrochen sein kann, worin **R⁹** für Wasserstoff oder einen C₁- - C₃-Alkylrest steht, oder
   c) einen unsubstituierten oder substituierten Aryl- oder Heteroarylrest oder
   d) einen unsubstituierten oder substituierten C₃- - C₁₀-Cycloalkylrest oder
   e) einen unsubstituierten oder substituierten C₄- - C₁₅-Cycloalkylalkylrest oder
   f) einen unsubstituierten oder substituierten C₇- - C₂₀-Aralkylrest oder
   g) einen unsubstituierten oder substituierten Heteroaryl-C₁- - -C₆--alkylrest oder
   h) einen unsubstituierten oder substituierten Aminoalkylrest oder einen Biphenylrest,
**X** ist vorzugsweise ein gerad- oder verzweigtkettiger C₁- - C₁₂-Alkylen-, -Alkenylen- oder -Alkinylenrest.

Bei **Y** kann es sich um eine direkte Bindung zwischen X und **Z** handeln. **Y** kann aber auch folgendes bedeuten:
a) eine SOₙ-**R¹⁰**-Gruppe, wobei n = 0,1 oder 2 ist und wobei **R¹⁰** eine direkte Bindung zwischen SOₙ und **Z** oder einen gerad- oder verzweigtkettigen C₁- - C₆-Alkylen-, -Alkenylen- oder -Alkinylenrest darstellt oder
b) die Gruppe **R¹¹** oder O-**R¹¹**, wobei **R¹¹** für
   i) einen gerad- oder verzweigtkettigen C₁- - C₅-Alkylen-, -Alkenylen- oder -Alkinylenrest steht oder für
   ii) einen unsubstituierten oder substituierten Arylrest oder Heteroarylrest steht oder für
   iii) einen unsubstituierten oder substituierten C₃- - C₁₀-Cycloalkylrest steht oder für
   iv) einen unsubstituierten oder substituierten C₄- - C₁₅-Cycloalkylalkylrest steht oder für
   v) einen unsubstituierten oder substituierten C₇- - C₂₀-Aralkylrest steht oder für
   vi) einen unsubstituierten oder substituierten Heteroaryl-C₁- - -C₆-alkylrest steht, oder
c) die Gruppierung CH = CF oder
d) die Gruppierung HN-C(O)-NH-**R¹²**,
   wobei **R¹²** für einen unsubstituierten oder substituierten Arylenrest steht und wobei **R¹²** an **Z** gebunden ist.
   **Z** stellt eine direkte Bindung zwischen **Y** und **E** oder einen gerad- oder verzweigtkettigen C₁- - C₉-Alkylen-, -Alkenylen- oder -Alkinylenrest dar, der teilweise oder vollständig fluoriert sein kann.
   **E** ist eine CF₃-Gruppe oder eine zumindest teilweise fluorierte Arylgruppe, insbesondere Phenylgruppe.

**Hal** steht insbesondere für Fluor.

**R³** kann Wasserstoff, Methyl, Ethyl, *n*-Propyl, *iso*-Propyl, *n*-Butyl, *iso*-Butyl und *tert*-Butyl, ein entsprechendes Alkanyol (Acetyl, Propionyl, Butanoyl) oder ein cyclischer Ether sein. **R³** steht insbesondere für Wasserstoff, CH₃, CH₃CO oder C₅H₁₀O.

**R^{17'}** und **R^{17"}** sind insbesondere Methyl, Ethyl, *n*-Propyl, *iso*-Propyl, *n*-Butyl, *iso*-Butyl und *tert*-Butyl, wobei **R^{17'}** zusätzlich auch Wasserstoff, Acetyl, Propionyl und Butanoyl sein kann, und wobei in diesem Falle die entsprechenden Isomeren einbezogen sein können. **R^{17"}** kann ausserdem Ethinyl, 1-Propinyl, 2-Propinyl, 1-Butinyl, 2-Butinyl und 3-Butinyl sowie Trifluormethyl, Pentafluorethyl, Heptafluorpropyl und Nonafluorbutyl sein, wobei in diesem Falle die entsprechenden Isomeren ebenfalls einbezogen sind. **R^{17'}** ist insbesondere Wasserstoff, CH₃ oder CH₃CO. **R^{17"}** steht vorzugsweise für Methyl, Ethinyl und Trifluormethyl.

**U** kann insbesondere ein gerad- oder verzweigtkettiger Alkylenrest und insbesondere ein Methylen-, Ethylen-, Propylen-, Butylen-, Pentylen-, Hexylen-, Heptylen-, Octylen-, Nonylen-, Decylen-, Undecylen-, Dodecylen- oder Tridecylenrest sein. Vorzugsweise steht **U** für (CH₂)p, wobei p eine ganze Zahl von 2 bis 10 ist. Insbesondere bevorzugt ist **U** ein Butylen-, Pentylen-, Hexylen- oder Heptylenrest. Ganz besonders bevorzugt ist **U** ein *n*-Butylenrest, d.h. in der Formel (CH₂)ₚ für **U** ist p = 4.

**V** steht insbesondere für CH₂. Damit kann die Gruppierung **U-V** in einer ganz bevorzugten Ausführungsform *n*-Pentylen sein.

**W** steht insbesondere für das Amin-N-Oxid **N**⁺(O⁻)(**R⁶**), wobei **R⁶** vorzugsweise Wasserstoff oder CH₂-**R⁷** ist, worin **R⁷** insbesondere für Wasserstoff oder Methyl oder Ethyl steht. **R⁶** ist damit vorzugsweise Wasserstoff oder ein C₁- - C₃-Alkylrest, also insbesondere ein Methyl-, Ethyl-, *n*-Propyl- oder *iso*-Propylrest. In einer besonders bevorzugten Ausführungsform stellt **W eine** N⁺(O⁻)(CH₃)-Gruppe dar (N-Methylamin-N-Oxid).

**X** steht vorzugsweise für (CH₂)_{q}, wobei q = 0 oder eine ganze Zahl von 1 bis 12 ist, also für eine direkte Bindung zwischen **W** und **Y** oder für einen geradkettigen oder verzweigten Methylen-, Ethylen-, Propylen-, Butylen-, Pentylen-, Hexylen-, Heptylen-, Octylen-, Nonylen-, Decylen-, Undecylen- oder Dodecylenrest. In einer insbesondere bevorzugten Ausführungsform ist **X** ein Ethylen-, *n*-Propylen-, *n*-Butylen-, *n*-Pentylen-, *n*-Hexylen-, *n-*Heptylen- oder *n*-Octylenrest.

**Y** kann insbesondere eine direkte Bindung zwischen **X** und **Z** darstellen. Falls dies der Fall ist, steht **X** für eine längere Alkylenkette, also insbesondere für *n*-Hexylen, *n*-Heptylen oder *n*-Octylen. **Y** kann in einer bevorzugten Ausführungsform auch eine SOₙ-Gruppe sein, wobei n = 0,1 oder 2 ist, also eine Sulfanyl-, eine Sulfinyl- oder eine Sulfonylgruppe. Falls Y eine SOₙ-Gruppe ist, stellt **X** eine eher kürzere Alkylenkette dar, insbesondere eine *n*-Propylkette.

Z ist vorzugsweise eine direkte Bindung zwischen **Y** und **E** oder ein gerad- oder verzweigtkettiger C₁- - C₇-Alkylenrest, der zumindest teilweise fluoriert sein kann. **Z** kann insbesondere ein Methylen-, Ethylen-, Propylen- oder Butylenrest sein, der zumindest teilweise fluoriert sein kann. **Z** ist insbesondere Difluormethylen oder ein geradkettiger Alkylenrest, der an einem Ende perfluoriert ist, also beispielsweise ein 1,1-Difluorethylen-, 1,1,2,2-Tetrafluor-n-propylen- oder 1,1,2,2,3,3-Hexafluor-n-butylenrest. Besonders günstig sind Alkylenreste, die lediglich zwei Fluoratome an einem endständigen C-Atom tragen, wobei diese CF₂-Gruppe an den Rest **E** gebunden ist. In diesem Falle ist die Seitenkette **SK** mit C₂F₅ terminiert.

**E** steht insbesondere für CF₃ oder für Pentafluorphenyl. Somit stellt die Gruppierung **Z-E** vorzugsweise eine der Gruppen, ausgewählt aus der Gruppe, umfassend C₂F₅, C₃F₇ und C₄F₉ sowie C₆F₅, dar.

Gemäß der vorliegenden Erfindung sind auch pharmakologisch verträgliche Säureadditionssalze sowie Ester der 17α-Alkyl-17β-oxy-estratriene umfasst. Bei den Additionssalzen handelt es sich um die entsprechenden Salze mit anorganischen und organischen Säuren. Als Additionssalze kommen insbesondere die Hydrochloride, Hydrobromide, Acetate, Citrate, Oxalate, Tartrate und Methansulfonate in Betracht. Falls **R³** und **R^{17'}** Wasserstoff sind, so dass ein 3,17β-Diol vorliegt, können auch die Ester dieser Hydroxyverbindungen gebildet werden. Diese Ester werden vorzugsweise mit organischen Säuren gebildet, wobei dieselben Säuren wie zur Bildung der Additionssalze in Frage kommen, nämlich insbesondere die Essigsäure, aber auch höhere Carbonsäuren, wie z. B. die Propion-, Butter-, Isobutter-, Valerian-, Isovalerian- oder die Pivalinsäure.

Die neuartigen 17α-Alkyl-17β-oxy-estratriene weisen mehrere chirale Zentren, beispielsweise auch an einem zum N-Oxid oxidierten N-Atom, auf. Daher gibt es jeweils mehrere stereoisomere Formen jeder Verbindung.

Besonders geeignete Verbindungen im erfindungsgemässen Sinne sind Estratriene mit der allgemeinen Formel I, nämlich
1) 11 β-Fluor-7α-{5-[methyl(7,7,8,8,9,9,9-heptafluornonyl)amino]pentyl}-17α-methylestra-1,3,5(10)-trien-3,17β-diol N-oxid
2) 11 β-Fluor-7α-{5-[methyl(8,8,9,9,10,10,10-heptafluordecyl)amino]pentyl}-17α-methylestra-1,3,5(10)-trien-3,17β-diol N-oxid
3) (RS)-11β-Fluor-7α-{5-[methyl(7,7,8,8,9,9,10,10,10-nonafluordecyl)amino]pentyl}-17α-methylestra-1,3,5(10)-trien-3,17β-diol N-oxid
4) 11 β-Fluor-7α-{5-[methyl(8,8,9,9,9-pentafluornonyl)amino]pentyl}-17α-methylestra-1,3,5(10)-trien-3,17β-diol N-oxid
5) 11 β-Fluor-7α-{5-[methyl(9,9,10,10,10-pentafluordecyl)amino]pentyl-17α-methylestra-1,3,5(10)-trien-3,17β-diol N-oxid

Physikalische Eigenschaften einiger dieser Verbindungen sind in Tabelle 1 angegeben.

Die erfindungsgemässen 17α-Alkyl-17β-oxy-estratriene unterscheiden sich von bekannten Verbindungen vor allem dadurch, dass in 11 α-Stellung ein Halogenatom und/oder in 17α-Stellung ein Alkylrest gebunden sind/ist. Ausserdem weisen die Verbindungen in der 7α-Seitenkette eine Amin-N-oxidgruppierung auf.

Im Gegensatz zu 3,17β-Dihydroxy-estratrienen, die in 17α-Stellung unsubstituiert sind, bilden sich aus den erfindungsgemässen 17α-Alkyl-17β-oxy-estratrienen praktisch keine Metaboliten. Metaboliten können ebenfalls biologisch aktiv sein. Es hat sich nämlich herausgestellt, dass die Estratrienderivate, die durch Oxidation der in 17β-Stellung gebundenen Hydroxygruppe entstehen, wobei ein 17-Oxoderivat entsteht, sehr starke biologische Aktivität aufweisen.

Durch Blockierung der 17α-Stellung durch einen Alkylrest, insbesondere durch eine C₁- - C₄-Alkylgruppe, wird diese Oxidationsreaktion unterbunden, so dass auch eine metabolische Vielfalt unterdrückt wird. Die als Wirkstoffe verwendeten erfindungsgemässen Estratriene weisen daher eine speziesunabhängige Wirksamkeit und Aktivität auf. Der Vorteil dieser Verbindungen besteht daher darin, dass die volle Wirksamkeit des Wirkstoffes in einer einzigen Verbindung realisiert wird.

Aus diesem Grunde ergeben sich Vorteile bei der Entwicklung von Arzneimitteln, denn mangels Bildung biologisch aktiver Metaboliten kann die Wirksamkeit bestimmten Strukturprinzipien einfacher zugeordnet werden, so dass eine zielgerichtete Wirkstoffsuche ermöglicht wird.

Ausserdem inhibieren die erfindungsgemässen 17α-Alkyl-17β-oxy-estratriene die Wirkung von Estradiol zu annähernd 100 %. Sie stellen daher Antiestrogene dar.

Zur Untersuchung der Wirksamkeit der erfindungsgemässen Verbindungen wurden in-vivo-Tests an der infantilen Ratte durchgeführt. Hierzu wurde das Uteruswachstum bei peroraler Gabe (p.o.) des Arzneimittels durchgeführt (Test auf antiestrogene Wirkung).

Das Prinzip dieser Methode besteht darin zu untersuchen, welchen Einfluss die Gabe von antiestrogen-wirkenden Verbindungen bei gleichzeitiger Applikation von Estrogenen hat. Bei Nagern reagiert der Uterus auf die Darreichung von Estrogenen nämlich mit einer Gewichtszunahme (sowohl durch Proliferation als auch durch Wassereinlagerung). Dieses Wachstum ist durch gleichzeitige Gabe antiestrogen-wirkender Verbindungen dosisabhängig zu hemmen.

Für die Tests wurden infantile weibliche Ratten mit einem Gewicht von 35 - 45 g bei Versuchsbeginn untersucht. Pro Dosis wurden fünf bis sechs Tiere getestet. Für die p.o. Applikation wurden die Substanzen in einem Teil Ethanol (E) gelöst und mit neun Teilen Erdnussöl (EÖ) aufgefüllt. Zur Eingewöhnung wurden die gerade von den Müttern abgesetzten jungen Ratten einen Tag vor Behandlungsbeginn geliefert und sofort mit Futter - auch in dem Tierkäfig - versorgt. Die Tiere wurden dann täglich einmal drei Tage lang in Kombination mit 0,5 µg Estradiolbenzoat (EB) behandelt. EB wurde immer subcutan (s.c.) appliziert, während die Testsubstanz p.o. verabreicht wurde. 24 Stunden nach der letzten Applikation wurden die Tiere gewogen, getötet und die Uteri entnommen. Von den präparierten Uteri wurden die Feuchtgewichte (ohne Inhalt) ermittelt. Es wurden folgende Kontrolluntersuchungen durchgeführt: Für eine negative Kontrolle wurden 0,2 ml einer E/EÖ-Mischung pro Tier undTag gegeben. Für eine positive Kontrolluntersuchungen wurden 0,5 µg EB/0,1 ml pro Tier und Tag appliziert.

Von den relativen Organgewichten (mg/100 g Körpergewicht) wurden für jede Gruppe die Mittelwerte mit Standardabweichung (X ± SD) sowie die Signifikanz der Unterschiede zur Kontrollgruppe (EB) im Dunnett-Test (p < 0,05) ermittelt. Die Hemmung (in %) gegenüber der EB-Kontrolle wurde mit einem Rechenprogramm ermittelt. Die relative Wirksamkeit der Prüfsubstanzen wurde durch eine Kovarianz- und Regressionsanalyse berechnet.

Untersuchungsergebnisse für ausgewählte Verbindungen sind in Tabelle 2 wiedergegeben. Dort sind Versuchsergebnisse für das Uteruswachstum bei gleichzeitiger Gabe von 0,5 µg EB/0,1 ml s.c. sowie peroraler Darreichung der antiestrogen wirkenden Verbindungen in einer Menge im Bereich von 0,03 mg/kg Körpergewicht und 0,3 mg/kg Körpergewicht wiedergegeben.

Aus Tabelle 2 ist erkennbar, dass die antiestrogene Wirkung nahezu bei 100 % liegt, wenn eine Dosierung von etwa 0,3 mg/kg bei peroraler Verabreichung gegeben wurde.

Die erfindungsgemässen Verbindungen sind ebenso wirksam wie oder sogar noch wirksamer als die entsprechenden Verbindungen, die in 17α-Stellung nicht substituiert sind. Gegenüber den in 17α-Stellung nicht substituierten Verbindungen weisen die erfindungsgemässen Estratriene ausserdem eine bessere Verträglichkeit auf, so dass letztere vorzuziehen sind. Die bessere Verträglichkeit ist insbesondere darauf zurückzuführen, dass eine Bildung von Metaboliten weitgehend eingeschränkt ist.

### Bestimmung der metabolischen Stabilität: in vitro 17β-HSD-Test

17β-HSD2 vermittelt die intestinale enzymatische Dehydrogenisierung einer OH-Gruppe in Position 17 des Sterangerüsts zu einer Keton-Gruppe.

Für diesen Versuch werden folgende Materialien verwendet:
Na-Phosphatpuffer: 100 mM Na₂HPO₄ x 2H₂O und100 mM NaH₂PO₄ x H₂O Testsubstanzlösung von
11 β-Fluor-7α-{5-[methyl(8,8,9,9,9-pentafluornonyl)amino]pentyl}-17α-methylestra-1,3,5(10)-trien-3,17β-diol (Verbindung 1, als ein Vertreter für die Verbindungen der allgemeinen Formel I) und
11β-Fluor-7α-{5-[methyl(8,8,9,9,9-pentafluornonyl)amino]pentyl-estra-1,3,5(10)-trien- 3,17β-diol (Verbindung 2): 15µM in MeOH (im Versuchsansatz 0,3µM).

Die Verbindungen 1 und 2 unterscheiden sich nur in der 17α-Methylgruppe, so dass der folgende Vergleichsversuch den Effekt der 17α-Methylgruppe auf die metabolische Stabilität dokumentiert, auch wenn die Verbindungen 1 und 2 nicht unter die allgemeine Formel I der vorliegenden Anmeldung fallen.

Kofaktor-Lösung: 2mL Glucose-6-Phoshat (160mM) / MgCl₂(80mM)-Mix werden zu 400 µL einer Glucose-6-Phoshat-Dehydrogenase-Lösung gegeben, anschließend werden 15,6 mg NADP und 13,4 mg NAD zugegeben.

Mikrosomen-Lösung: Intestinale Mikrosomen (InVitroTechnologies; Proteingehalt: 24mg/mL; CYP450-Gehalt: 0,058nmol/mg Protein)
Im Wasserbad bei 37°C aufgetaut (~60sec) und auf eine Konzentration von 5mg/mL Protein mit Na-Phosphatpuffer verdünnt.

Es werden jeweils 170 µL/well des Puffers und 5µL/well der Testsubstanzlösungen in die entsprechenden Wells vorgelegt, wobei für jeden Messzeitpunkt (0, 10, 20, 30, 45 und 60 Minuten) Doppelwerte angesetzt werden.
Zu den 0 Minuten-Werten wird jeweils 250µL eiskaltes MeOH gegeben. Unverzüglich danach werden zu allen Wells 25µL Mikrosomen-Lösung und 50µL Kofaktor-Lösung gegeben. Die Proben der 0-Minuten-Werte werden ohne Inkubation bei ~ -20°C für ca. 24h gelagert. Die anderen Proben werden jeweils für 10, 20, 30, 45 und 60 Minuten bei 37°C inkubiert und durch die Zugabe von jeweils 250µL eiskaltem MeOH nach diesen Zeitpunkten wird die Dehydrogenisierungsreaktion gestoppt. Die Proben werden bis zur Vermessung per LC/MS/MS bei ~ -20°C ca. 24h gelagert und vor der Analyse bei 3000 Upm zentrifugiert, wobei der Überstand vermessen wird.
Die per LC/MS/MS gemessenen Konzentrationen an den Prüfsubstanzen und das entstehende 17 Keton-Produkt werden in Fig. 2a - 2f wiedergegeben.

Verbindung 1 ist metabolisch stabil in intestinalen aber nicht in Lebermikrosomen, was darauf hindeutet, dass in beiden Geweben unterschiedliche Phase-1 Reaktionen stattfinden. In keinem der Gewebe tritt jedoch das putative Produkt der 17ßHSD-Reaktion, 11 β-Fluor-7α-{5-[methyl(8,8,9,9,9-pentafluornonyl)amino]pentyl}-estra-1,3,5(10)-trien-3-ol-17-on, Verbindung 3, auf. Im Gegensatz dazu wird Verbindung 2, die keine 17 Methylgruppe besitzt, in intestinalen Mikrosomen abgebaut, wobei das entsprechende 17-Keton entsteht. Demzufolge kann die hohe metabolische Stabilität von Verbindung 1 durch die Blockade der 17ßHSD Reaktion erklärt werden, die durch eine 17β-Methylgruppe vollständig unterbunden wird. Es ist deshalb davon auszugehen, dass eine Alkyl-, beispielsweise eine Methylgruppe, oder auch eine Alkenyl- oder Alkinyl-, beispielsweise eine Ethinylgruppe, in Nachbarschaft zur 17-OH-Gruppe überraschenderweise deren intestinale (im Gegensatz zur hepatischen) Oxidation zum Keton verhindert, was eine höhere orale Bioverfügbarkeit zur Folge haben sollte.

Die erfindungsgemässen Verbindungen zeichnen sich also desweiteren durch eine ausserordentlich hohe Bioverfügbarkeit aus, so dass durch die Applikation der erfindungsgemässen Verbindungen beim betroffenen Patienten hohe Serumspiegel erreicht werden können. In Zusammenhang mit der schon erwähnten hohen Verträglichkeit lässt sich so eine erfolgreiche und sichere Therapie durchführen, weil es mit den erfindungsgemässen Verbindungen gelingt, einen Serumspiegel der wirksamen Verbindung einzustellen, der einen ausreichenden Abstand zum Wirkspiegel entsprechenden Verbindung aufweist. Mit Wirkspiegel ist die Serumkonzentration des Wirkstoffes gemeint, die zur Erreichung des gewünschten Effektes in der jeweiligen Indikation mindestens erforderlich ist.

Die erfindungsgemässen 17α-Alkyl-17β-oxy-estratriene mit der allgemeinen Formel I sind insbesondere zur Herstellung von Arzneimitteln geeignet. Die Erfindung betrifft daher ausserdem pharmazeutische Präparate, die neben mindestens einem 17α-Alkyl-17β-oxy-estratrien mit der allgemeinen Formel I, das die Substituenten **Hal, R³, R^{17'}, R^{17"}, U, V, W, X, Y, Z** und **E** gemäß vorstehenden Definitionen hat, mindestens einen pharmazeutisch verträglichen Träger enthält. Die pharmazeutischen Präparate bzw. Zusammensetzungen gemäß der Erfindung werden mit üblichen festen oder flüssigen Trägerstoffen oder Verdünnungsmitteln und üblichen pharmazeutischen und technischen Hilfsstoffen entsprechend der gewünschten Applikationsart mit einer geeigneten Dosierung in an sich bekannter Weise hergestellt. Bevorzugte Zubereitungen bestehen in einer Darreichungsform, die zur oralen, enteralen oder parenteralen, beispielsweise *i*.*p*. (intraperitonealen), *i*.*v*. (intravenösen), *i*.*m*. (intramuskulären), oder perkutanen, Applikation geeignet ist. Solche Darreichungsformen sind beispielsweise Tabletten, Filmtabletten, Dragees, Pillen, Kapseln, Pulver, Cremes, Salben, Lotionen, Flüssigkeiten, wie Sirupe, Gele, injizierbare Flüssigkeiten, beispielsweise zur *i*.*p*., *i.v., i*.*m*. oder perkutanen Injektion usw. Weiterhin sind auch Depotformen, wie implantierbare Zubereitungen, sowie Suppositorien geeignet. Dabei geben die einzelnen Zubereitungen die erfindungsgemässen Estratriene je nach deren Art allmählich oder die gesamte Menge in kurzer Zeit an den Körper ab.

Zur oralen Verabreichung können Kapseln, Pillen, Tabletten, Dragees und Flüssigkeiten oder andere bekannte orale Darreichungsformen als pharmazeutische Präparate eingesetzt werden. In diesem Falle können die Arzneimittel in der Weise formuliert sein, dass sie die Wirkstoffe entweder in kurzer Zeit freisetzen und an den Körper abgeben oder eine Depotwirkung aufweisen, so dass eine länger anhaltende, langsame Zufuhr von Wirkstoff zum Körper erreicht wird. Die Dosierungseinheiten können neben dem mindestens einen Estratrien einen oder mehrere pharmazeutisch verträgliche Träger enthalten, beispielsweise Stoffe zur Einstellung der Rheologie des Arzneimittels, oberflächenaktive Stoffe, Lösungsvermittler, Mikrokapseln, Mikropartikel, Granulate, Verdünner, Bindemittel, wie Stärke, Zucker, Sorbit und Gelatine, ferner Füllstoffe, wie Kieselsäure und Talkum, Gleitmittel, Farbstoffe, Duftstoffe und andere Stoffe.

Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog zu den Tabletten hergestellten Kernen mit üblicherweise in Dragéeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Die erfindungsgemässen Estratriene können auch in Form einer Lösung formuliert werden, die für die orale Verabreichung bestimmt ist und die neben dem aktiven Estratrien als Bestandteile ein pharmazeutisch verträgliches Öl und/oder eine pharmazeutisch verträgliche lipophile, oberflächenaktive Substanz und/oder eine pharmazeutisch verträgliche hydrophile, oberflächenaktive Substanz und/oder ein pharmazeutisch verträgliches wassermischbares Lösungsmittel enthält.

Um eine bessere Bioverfügbarkeit der erfindungsgemässen Wirkstoffe zu erreichen, können die Verbindungen auch als Cyclodextrinchlatrate formuliert werden. Hierzu werden die Verbindungen mit α-, β- oder γ-Cyclodextrin oder deren Derivaten umgesetzt.

Falls Cremes, Salben, Lotionen und äusserlich anwendbare Flüssigkeiten eingesetzt werden sollen, müssen diese so beschaffen sein, dass die erfindungsgemässen Verbindungen dem Körper in ausreichender Menge zugeführt werden. In diesen Darreichungsformen sind Hilfsstoffe enthalten, beispielsweise Stoffe zur Einstellung der Rheologie der Arzneimittel, oberflächenaktive Mittel, Konservierungsmittel, Lösungsvermittler, Verdünner, Stoffe zur Erhöhung der Permeationsfähigkeit für die erfindungsgemässen Estratriene durch die Haut, Farbstoffe, Duftstoffe und Hautschutzmittel, wie Konditionierer und Feuchteregulatoren. Zusammen mit den erfindungsgemässen Verbindungen können auch andere Wirkstoffe in dem Arzneimittel enthalten sein [Ullmanns Enzyklopädie der technischen Chemie, Band 4 (1953), Seiten 1 - 39; J. Pharm. Sci., 52, 918 ff. (1963); H. v.Czetsch-Lindenwald, Hilfsstoffe für Pharmazie und angrenzende Gebiete; Pharm. Ind., 2, 72 ff (1961); Dr. H. P. Fiedler, Lexikon der Hilfsstoffe für Pharmazie , Kosmetik und angrenzende Gebiete, Cantor AG, Aulendorf/Württ., 1971].

Die erfindungsgemässen Substanzen können auch in geeigneten Lösungen, wie beispielsweise physiologischer Kochsalzlösung, als Infusions- oder Injektionslösung zur Anwendung kommen. Für die parenterale Applikation können die Wirkstoffe in einem physiologisch verträglichen Verdünnungsmittel gelöst oder suspendiert sein. Als Verdünnungsmittel sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Zur Formulierung eines injizierbaren Präparats kann ein beliebiger flüssiger Träger verwendet werden, in dem die erfindungsgemässen Verbindungen gelöst oder emulgiert sind. Diese Flüssigkeiten enthalten häufig auch Stoffe zur Regulation der Viskosität, oberflächenaktive Stoffe, Konservierungsstoffe, Lösungsvermittler, Verdünner und weitere Zusatzstoffe, mit denen die Lösung isotonisch eingestellt wird. Zusammen mit den Estratrienen können auch andere Wirkstoffe verabreicht werden.

Die erfindungsgemässen Estratriene können auch in Form einer Depotinjektion oder eines Implantatpräparats, beispielsweise subkutan, angewendet werden. Derartige Präparate können so formuliert sein, dass eine verzögerte Wirkstoff-Freigabe ermöglicht wird. Hierzu können bekannte Techniken eingesetzt werden, beispielsweise sich auflösende oder mit einer Membran arbeitende Depots. Implantate können als inerte Materialien beispielsweise biologisch abbaubare Polymere oder synthetische Silikone, beispielsweise Silikonkautschuk, enthalten. Die Estratriene können ferner zur perkutanen Verabreichung beispielsweise in ein Pflaster eingearbeitet werden.

Es ist auch möglich, die erfindungsgemässen Substanzen in ein transdermales System einzuarbeiten und sie damit transdermal zu applizieren.
Um einen verbesserten transdermalen Hautfluß zu erreichen, der therapeutisch wirksame Blutspiegel erzeugt, können die erfindungsgemässen Verbindungen auch analog wie es für andere Antiestrogene in der WO 01/76608 beschrieben ist, in Transdermalsysteme eingearbeitet werden. Diese Transdermalsysteme zeichnen sich durch ein spezielles Verhältnis von 2 Penetrationsverstärkern, insbesondere Laurinsäue und Propylenglycol, aus.

Die Dosierung der erfindungsgemässen Substanzen der allgemeinen Formel I wird vom behandelnden Arzt bestimmt und hängt unter anderem von der verabreichten Substanz, dem Verabreichungsweg, der zu behandelnden Erkrankung und von der Schwere der Erkrankung ab. Die zu verabreichende Menge der Verbindungen schwankt innerhalb eines weiten Bereichs und kann jede wirksame Menge abdecken. In Abhängigkeit vom zu behandelnden Zustand und von der Art der Verabreichung kann die Menge der verabreichten Verbindung 0,1 - 25 mg/kg Körpergewicht, vorzugsweise 0,5 - 5 mg/kg Körpergewicht, je Tag betragen. Beim Menschen entspricht dies einer täglichen Dosis von 5 - 1250 mg. Die bevorzugte tägliche Dosierung beim Menschen beträgt 50 - 200 mg. Dies gilt insbesondere für die Tumortherapie. Die Dosis kann als einmal zu verabreichende Einzeldosis oder unterteilt in zwei oder mehrere Tagesdosen gegeben werden.

Die Verbindungen der allgeminen Formel I stellen, wie schon erwähnt, Verbindungen mit sehr starker antiestrogener Wirksamkeit dar.
Die Verbindungen eignen sich zur Therapie von estrogen-abhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Prostatahyperplasie, anovulatorische Infertilität und Melanom.
Die Verbindungen der allgemeinen Formel I können auch als Komponente in den u. a. in der EP 346 014 B1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen.
Die Verbindungen der allgemeinen Formel I können gemeinsam mit Antigestgenen (kompetitiven Progesteronantagonisten) zur Behandlung hormonabhängiger Tumoren verwendet werden (EP 310 542 A).
Weitere Indikationen, in denen die Verbindungen der allgemeinen Formel I zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530).
Außerdem können die Verbindungen der allgemeinen Formel I zur Herstellung von Medikamenten zur Behandlung der Endometriose verwendet werden.
Ferner kann man die Verbindungen der allgemeinen Formel I zur Herstellung pharmazeutischer Zusammensetzungen für die männliche und weibliche Fertilitätskontrolle einsetzen (männliche Fertilitätskontrolle: DE 195 10 862.0 A).

Die erfindungsgemässen Estratriene können analog zu bekannten Verfahren hergestellt werden:

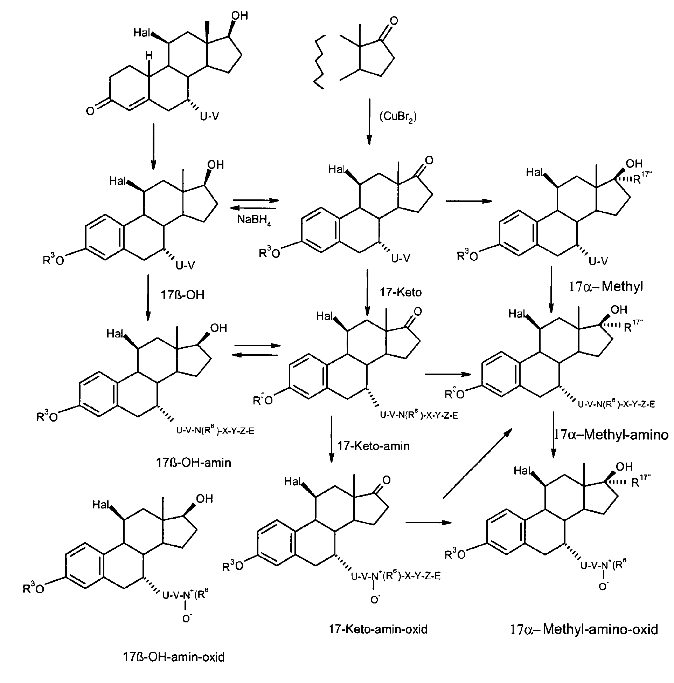

In Figur 1 ist ein Reaktionsschema wiedergegeben, nach dem die erfindungsgemässen Verbindungen hergestellt werden können. Die erfindungsgemässen 17α-Alkyl-17β-oxy-estratriene sind in diesem Schema mit dem Begriff "17α-Methyl-amin-oxid" bezeichnet. Dagegen weisen die Verbindungen mit der Bezeichnung "17α-Methyl" in 7α-Stellung eine Seitenkette ohne Amingruppierung auf. Die Verbindungen, die in 17β-Stellung eine Hydroxy- oder Alkoxygruppe, in 17α-Stellung eine Alkylgruppe sowie eine 7α-Seitenkette mit Amingruppierung tragen, werden mit "17α-Methyl-amin" bezeichnet. In entsprechender Weise sind die Verbindungen, bezeichnet mit "17α-Methyl-amin-oxid", die erfindungsgemässen Amin-N-oxide der vorbezeichneten "17α-Methyl-amin"-Verbindungen. Falls R³≠ H ist, wird eine Veretherung mit einem Reagenz R³ vorgenommen, worin X eine Fluchtgruppe bedeutet.
Ferner sind Verbindungen, die mit "17β-OH" bezeichnet sind, Estratriene, die in 17β-Stellung eine Hydroxy- oder Alkoxygruppe, jedoch weder eine 17α-Alkyl-, noch eine Amingruppierung in der Seitenkette in 7α-Stellung aufweisen. Verbindungen, die mit "17-Keto" bezeichnet sind, sind Estratriene, die in 17-Stellung eine Oxogruppe tragen, jedoch keine Amingruppierung in der Seitenkette in 7α-Stellung. Die weiteren Verbindungen, die mit "17β-OH-amin", mit"17-Keto-amin", mit"17β-OH-amin-oxid" und mit "17-Keto-amin-oxid" bezeichnet sind, weisen entsprechende Substitutionsmuster auf.

Grundsätzlich können alle aufgeführten Verbindungen, ausgehend von der 17-Oxoverbindung, hergestellt werden. Die Herstellung der 17-Oxoverbindungen ist beispielsweise in WO 99/33855 A1 exemplarisch beschrieben. Andere Derivate als die ausdrücklich in diesem Dokument offenbarten Verbindungen mit demselben Substitutionsmuster können analog hergestellt werden. In gleicher Weise können die erfindungsgemässen Estratriene auch, ausgehend von den 17β-Hydroxy- oder 17β-Alkoxyverbindungen ("17β-OH"), hergestellt werden. Die Herstellung dieser Derivate ist ebenfalls beispielsweise in WO 99/33855 A1 angegeben. In gleicher Weise ist in diesem Dokument auch die Herstellung der 17β-Hydroxy- oder 17β-Alkoxyverbindungen sowie der 17-Oxoverbindungen mit Amingruppierung in der Seitenkette in 7α-Stellung offenbart. Soweit die Herstellung der Ausgangsverbindungen nicht beschrieben wird, sind die Ausgangsverbindungen bekannt und käuflich, oder die Verbindungen werden analog zu den beschriebenen Verfahren synthetisiert. Nachfolgend wird die Herstellung einiger Vorstufen, Zwischenprodukte und Produkte exemplarisch beschrieben.

Bei der Herstellung der erfindungsgemässen Substanzen bedient man sich beispielsweise folgender Verfahren (siehe hierzu auch EP 0138 504 B1; WO 97/45441 A1; WO 98/07740 A1; WO 99/33855 A1):

Die erfindungsgemässen 17α-Alkyl-17β-oxy-estratriene können, ausgehend von den entsprechenden 17β-Oxy-estratrienen ("17β-OH"), hergestellt werden. Die Synthese dieser Ausgangssubstanzen ist beispielsweise auch in WO 97/45441 A1 und WO 98/07740 A1 beschrieben. Die Seitenkette in 7α-Stellung kann beispielsweise nach der in WO 98/07740 A1 angegebenen Vorgehensweise aufgebaut werden.

Anschließend kann die entstandene 17β-Hydroxy- oder 17β-Alkoxyverbindung mit Amingruppierung in der Seitenkette in 7α-Stellung durch Oxidation zur entsprechenden 17-Oxoverbindung oxidiert werden ("17-Keto-amin"). Hierzu können übliche Oxidationsmittel, beispielsweise Chrom(VI)-Verbindungen (Jones-Oxidation), Salpetersäure, Braunstein, Selendioxid und SO₃ in Pyridin eingesetzt werden. Die Ketone können auch durch katalytische Dehydrierung mit metallischem Kupfer, Silber, Kupferchromat und Zinkoxid bei erhöhter Temperatur oder durch Dehydrierung mit Ketonen, beispielsweise Cyclohexanon, durch Oppenauer-Oxidation hergestellt werden. Falls die Seitenkette reduzierende Gruppen enthält, etwa S- oder SO-Gruppen, so können diese nach einer Überoxidation gegebenenfalls wieder selektiv reduziert werden

In einer weiteren Verfahrensvariante können die 17β-Oxy-estratriene ohne Aminpruggierung in der 7α-Seitenkette direkt zu den 17-Oxo-estratrienen ("17-Keto") oxidiert und diese danach in bekannter Weise in der 7α-Seitenkette aminiert werden.

Anschliessend kann eine Alkylgruppe in 17α-Stellung eingeführt werden. Hierzu können übliche nukleophile Alkylierungsreagentien verwendet werden, beispielsweise Grignard-Regentien oder Alkyllithium-Verbindungen. Bei dieser Reaktion entstehen die gewünschten 17α-Alkyl-17β-oxy-estratriene ("17α-Methyl", wenn von den entsprechenden 17β-Hydroxyestratrienen ohne Amingruppierung in der Seitenkette in 7α-Stellung ["17β-OH"] ausgegangen wird, oder "17α-Methyl-amin", wenn von den entsprechenden 7-Hydroxyestratrienen mit Amingruppierung in der Seitenkette in 17α-Stellung ["17β-OH-amin"] ausgegangen wird). Ausserdem können die als Zwischenprodukte erhaltenen 17-Oxo-estratriene zunächst ist bekannter Weise alkyliert und danach in der 7α-Seitenkette aminiert werden.

Zur Herstellung der erfindungsgemäßen Amin-N-oxidverbindungen ("17β-OH-amin-oxid" oder "17-Keto-amin-oxid" oder "17α-Methyl-amin-oxid") werden die entsprechenden Estratriene mit Amingruppierung in der 7α-Seitenkette ("17β-OH-amin" bzw. "17-Keto-amin" bzw. "17α-Methyl-amin") oxidiert, beispielsweise mit Wasserstoffperoxid. Bei dieser Reaktion wird die sekundäre OH-Gruppe in 17β-Stellung nicht oxidiert.

In einer alternativen Vorgehensweise zur Herstellung der erfindungsgemässen 17α-Alkyl-17β-oxy-estratriene dienen die vorerwähnten 17β-Hydroxy-estratriene mit Amingruppierung in der Seitenkette in 7α-Stellung ("17β-OH-amin") ebenfalls als Ausgangssubstanzen.

Diese werden zuerst zu den entsprechenden Amin-N-oxidverbindungen ("17β-OH-amin-oxid") umgesetzt, wobei, wie vorstehend angegeben, übliche Oxidationsmittel, beispielsweise Wasserstoffperoxid, verwendet werden.

Anschliessend können die gebildeten Amin-N-oxidverbindungen ("17β-OH-amin-oxid")zum entsprechenden Keton ("17-Keto-amin-oxid") oxidiert werden, wobei dieselben Oxidationsmittel, wie oben angegeben, eingesetzt werden können. Dabei entstehen die 17-Oxoverbindungen mit Amin-N-oxidgruppierung in der 7α-Seitenkette.

Zur Herstellung der erfindungsgemässen 17α-Alkyl-17β-oxy-estratriene wird die Ketogruppe wiederum gemäss vorstehender Vorschrift mit geeigneten nukleophilen Alkylierungsreagentien umgesetzt. Dabei entstehen die 17α-Alkyl-17β-oxy-estratriene mit Amin-N-oxidgruppierung in der 7α-Seitenkette ("17α-Methyl-amin-oxid").

Zur Herstellung der erfindungsgemässen 17α-Alkyl-17β-oxy-estratriene werden also u.a. auch Zwischenprodukte mit folgender allgemeiner Formel II gebildet, die ebenfalls Gegenstand der vorliegenden Erfindung sind:

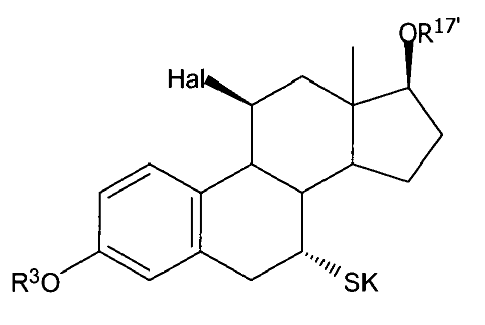

Darin sind wiederum:
**Hal** = F oder Cl, wobei dieser Rest in 11 β-Position an das Estratriengrundgerüst gebunden ist,
**R³** = Wasserstoff, C₁- - C₄-Alkyl, C₁- - C₄-Alkanoyl oder ein cyclischer C₃- - C₇-Ether mit einem O-Atom,
**R^{17'}** = Wasserstoff, C₁- - C₄-Alkyl und C₁- - C₄-Alkanoyl, wobei **R^{17'}** in 17β-Position an das Estratriengrundgerüst gebunden ist, und
**SK** = **U-V-W-X-Y-Z-E,** wobei diese Gruppierung über **U** in 7α-Position an das Estratriengerüst gebunden ist und wobei **U, V, X, Y, Z** und **E** die weiter oben angegebenen Bedeutungen haben und **W** für N⁺(O⁻)(**R⁶**)- oder für einen Azolidinylen-N-Oxid-Ring steht, wobei der Azolidinylen-N-Oxid-Ring mindestens ein C-Atom der Gruppierung **X** einschliesst, wobei **R⁶** im übrigen die weiter oben angegebene Bedeutung hat.

Besonders bevorzugte 17α-Alkyl-17β-Oxy-estratriene mit Amin-N-oxidgruppierung in der 7α-Seitenkette mit der allgemeinen Formel II sind die folgenden Verbindungen:
X1) 11 β-Fluor-7α-{5-[methyl(8,8,9,9,9-pentafluornonyl)amino]pentyl}estra-1,3,5(10)-trien-3,17β-diol N-oxid
X2) 11 β-Fluor-7α-[5-(methyl{3-[(2,3,4,5,6-pentafluorphenyl)sulfanyl]propyl}amino)pentyl]estra-1,3,5(10)-trien-3,17β-diol N-oxid
X3) 11β-Fluor-7α-[5-(methyl{3-[(4,4,5,5,5-pentafluorpentyl)sulfanyl]propyl}amino)pentyl]estra-1,3,5(10)-trien-3,17β-diol N-oxid
X4) 11 β-Fluor-7α-[5-(methyl{3-[(4,4,5,5,5-pentafluorpentyl)sulfinyl]propyl}amino)pentyl]estra-1,3,5(10)-trien-3,17β-diol N-oxid
X5) 11 β-Fluor-7α-{5-[methyl(7,7,8,8,9,9,10,10,10-nonafluordecyl)amino]pentyl}estra-1,3,5(10)-trien-3,17β-diol N-oxid
X6) (S)-11β-Fluor-7α-{5-[methyl(7,7,8,8,9,9,10,10,10-nonafluordecyl)amino]pentyl}-17α-methylstra-1,3,5(10)-trien-3,17β-diol N-oxid
X7) (R)-11β-Fluor-7α-{5-[methyl(7,7,8,8,9,9,10,10,10-nonafluordecyl)amino]pentyl}-17α-methylestra-1,3,5(10)-trien-3,17β-diol N-oxid
X8) 11 β-Fluor-7α-{5-[methyl(9,9,10,10,10-pentafluordecyl)amino]pentyl}estra-1,3,5(10)-trien-3,17β-diol N-oxid.

Physikalische Eigenschaften dieser Verbindungen sind in Tabelle 3 angegeben.

Weiterhin sind auch die bei der Herstellung der erfindungsgemässen 17α-Alkyl-17β-oxy-estratriene gebildeten 17-Oxo-estratriene mit Amin-N-oxidgruppierung in der 7α-Seitenkette als Zwischenprodukte Gegenstand der vorliegenden Erfindung. Diese Verbindungen haben die allgemeine Formel III:

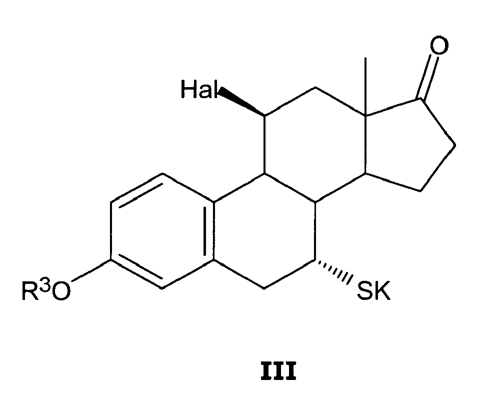

Darin bedeuten:
**Hal** = F oder CI, wobei dieser Rest in 11 β-Position an das Estratriengrundgerüst gebunden ist,
**R³** = Wasserstoff, C₁- - C₄-Alkyl, C₁- - C₄-Alkanoyl oder cyclischer ein C₃- - C₇-Ether mit einem O-Atom, und
**SK = U-V-W-X-Y-Z-E** ist, wobei diese Gruppierung über **U** in 7α-Position an das Estratriengrundgerüst gebunden ist und wobei **U, V, X, Y, Z** und **E** die weiter oben angegebenen Bedeutungen haben und **W** für eine N⁺(O⁻)(**R⁶**)-Gruppe oder für einen Azolidinylen-N-Oxid-Ring steht, wobei der Azolidinylen-N-Oxid-Ring mindestens ein C-Atom der Gruppierung **X** einschliesst, wobei **R⁶** ebenfalls die weiter oben angegebene Bedeutung hat.

Besonders bevorzugte 17-Oxo-estratriene mit einer Amin-N-oxidgruppierung in der 7α-Seitenkette mit der allgemeinen Formel III sind die folgenden Verbindungen:
- Y1: 11 β-Fluor-7α-[5-(methyl{3-[(4,4,5,5,5-pentafluorpentyl)sulfinyl]propyl}amino)pentyl]estra-1,3,5(10)-trien-3-ol-17-on N-oxid
- Y2: 11 β-Fluor-7α-[5-(methyl{3-[(4,4,5,5,5-pentafluorpentyl)sulfanyl]propyl}amino)pentyl]estra-1,3,5(10)-trien-3-ol-17-on N-oxid
- Y3: 11 β-Fluor-7α-{5-[methyl(7,7,8,8,9,9,10,10,10-nonafluordecyl)amino]pentyl}estra-1,3,5(10)-trien-3-ol-17-on N-oxid

Physikalische Eigenschaften dieser Verbindungen sind in Tabelle 4 angegeben.

Bei den Verbindungen der allgemeinen Formel II und den Verbindungen der allgemeinen Formel III handelt es sich ebenfalls um Verbindungen mit antiestrogener Wirksamkeit. Sie können daher prinzipiell in den vorstehend für die Verbindungen der allgemeinen Formel I angegebenen Indikationsgebieten eingesetzt werden.

Nachfolgend werden die Verfahrensschritte zur Herstellung der erfindungsgemässen Verbindungen näher beschrieben.
Die in den folgenden Beispielen genannten "Amin"-verbindungen sind nur zur Erklärung der Synthese erwähnt. Sie sind nicht Gegenstand der vorliegenden Erfindung.

### Verfahrensvariante 1.1

(Herstellung von 17-Oxo-estratrienen mit Amin-N-oxidgruppierung in der Seitenkette, ausgehend von 17β-Hydroxy-estratrienen mit Amingruppierung in der Seitenkette über die entsprechenden 17-Oxoestratriene):
**a) 11β-Fluor-7α-{5-[methyl(7,7,8,8,9,9,10,10,10-nonafluordecyl)amino]pentyl}estra-1,3,5(10)-trien-3-ol-17-on** (Drehwert α_{D} dieser Verbindung (Nr. Z14) ist in Tabelle 5 angegeben)
   Zu einer Lösung von 1,23 g Pyridinschwefeltrioxidkomplex in 10 ml getrocknetem Dimethylsulfoxid tropft man bei 10°C 1,5 ml Ethyldiisopropylamin. Dann gibt man 1,72 g 11 β-Fluor-7α-{5-[methyl(7,7,8,8,9,9,10,10,10-nonafluordecyl)amino]pentyl}estra-1,3,5(10)-trien-3,17β-diol (Verbindung Nr. Z9) sowie weitere 10 ml getrocknetes Dimethylsulfoxid zu und rührt 30 Minuten bei Raumtemperatur. Dann wird mit Essigester verdünnt, mit gesättigter Natriumhydrogencarbonatlösung, Wasser und Natriumchloridlösung gewaschen, über Natriumsulfat getrocknet, im Vakuum zur Trockne eingeengt und an Kieselgel mit Dichlormethan/Methanol chromatographiert. Man erhält 11 β-Fluor-7α-{5-[methyl(7,7,8,8,9,9,10,10,10-nonafluordecyl)amino]pentyl}estra-1,3,5(10)-trien-3-ol-17-on. [α]_{D} = +58,2° in Chloroform.
**b) 11β-Fluor-7α-{5-[methyl(7,7,8,8,9,9,10,10,10-nonafluordeeyl)amino]pentyl}estra-1,3,5(10)-trien-3-01-17-on N-oxid**
   Eine Lösung von 0,5 g 11β-Fluor-7α-{5-[methyl(7,7,8,8,9,9,10,10,10-nonafluordecyl)amino]pentyl}estra-1,3,5(10)-trien-3-ol-17-on in 11 ml Methanol und 11ml Chloroform wird mit 3,5 ml 30%iger Wasserstoffperoxidlösung versetzt und fünf Tage bei Raumtemperatur gerührt. Dann wird mit Natriumthiosulfat versetzt, auf Wasser gegeben, dreimal mit Dichlormethan extrahiert, neutralgewaschen, über Natriumsulfat getrocknet, im Vakuum zur Trockne eingeengt und an Kieselgel mit Dichlormethan/Methanol chromatographiert. Man erhält 401 mg 11β-Fluor-7α-{5-[methyl(7,7,8,8,9,9,10,10,10-nonafluordecyl)amino]pentyl}estra-1,3,5(10)-trien-3-ol-17-on N-oxid als Feststoff vom Schmelzpunkt 84-86°C; [α]_{D} = +53,6° in Chloroform.
**c) 11β-Fluor-7α-{5-[methyl(7,7,8,8,9,9,10,10,10-nonafluordeeyl)amino]pentyl}17β-methylestra-1,3,5(10)-trien-3,17-diol N-oxid**
   Eine Suspension von 2,3 g Cer-III-chlorid in 23 ml Tetrahydrofuran wird bei 0°C mit 3,19 ml einer 3-molaren Methylmagnesiumbromidlösung in Diethylether versetzt und 30 Minuten gerührt. Dazu tropft man eine Lösung von 250 mg 11β-Fluor-7α-{5-[methyl(7,7,8,8,9,9,10,10,10-nonafluordecyl)amino]pentyl}estra-1,3,5(10)-trien-3-ol-17-on N-oxid in 5 ml Tetrahydrofuran und rührt anschließend 24 Stunden bei Raumtemperatur, versetzt bei 0°C mit 10 ml Ammoniumchloridlösung, extrahiert mit Essigester, wäscht mit Wasser, trocknet mit Natriumsulfat, engt im Vakuum ein, nimmt mit 5 ml Methanol und 5 ml Chloroform auf, versetzt mit 2 ml 30%iger Wasserstoffperoxidlösung, versetzt und rührt 5 Tage bei Raumtemperatur. Dann wird mit Natriumthiosulfat versetzt, auf Wasser gegeben, dreimal mit Dichlormethan extrahiert, neutralgewaschen, über Natriumsulfat getrocknet, im Vakuum zur Trockne eingeengt und an Kieselgel mit Dichlormethan / Methanol chromatographiert. Man erhält 165 mg 11 β-Fluor-7α-{5-[methyl(7,7,8,8,9,9,10,10,10-nonafluordecyl)amino]pentyl}-17α-methylestra-1,3,5(10)-trien-3,17β-diol N-oxid vom Schmelzpunkt 122°C.

### Verfahrensvariante 1.2:

(Herstellung von 17-Oxo-estratrienen mit Amin-N-oxidgruppierung in der Seitenkette, ausgehend von 17β-Hydroxy-estratrienen mit Amingruppierung in der Seitenkette über die entsprechenden 17β-Hydroxy-estratriene mit Amin-N-oxidgruppierung in der Seitenkette):
**a) 11β-Fluor-7α-{5-[methyl(7,7,8,8,9,9,10,10,10-nonafluordecyl)amino]pentyl}-estra-1,3,5(10)-trien-3,17-diol N-oxid**
   Eine Lösung von 50 g 11β-Fluor-7α-{5-[methyl(7,7,8,8,9,9,10,10,10-nonafluordecyl)amino]pentyl}-estra-1,3,5(10)-trien-3,17β-diol in 500 ml Methanol und 500 ml Chloroform wird mit 7,3 g Natriumhydrogencarbonat sowie 45 ml 30%iger Wasserstoffperoxidlösung versetzt und 3 Tage bei Raumtemperatur gerührt. Dann wird mit Natriumthiosulfat versetzt, auf Wasser gegeben, dreimal mit Dichlormethan extrahiert, neutralgewaschen, über Natriumsulfat getrocknet, im Vakuum zur Trockne eingeengt und aus Diethylether ausgerührt. Man erhält 48,3 g 11 β-Fluor-7α-{5-[methyl(7,7,8,8,9,9,10,10,10-nonafluordecyl)amino]pentyl}-estra-1,3,5(10)-trien-3,17β-diol N-oxid vom Schmelzpunkt 131,7°C.
**b) 11β-Fluor-7α-{5-[methyl(7,7,8,8,9,9,10,10,10-nonafluordecyl)amino]pentyl}-estra-1,3,5(10)-trien-3-ol-17on N-oxid**
   Zu einer Lösung von 1,23 g Pyridinschwefeltrioxidkomplex in 10 ml getrocknetem Dimethylsulfoxid tropft man bei 10°C 1,5 ml Ethyldiisopropylamin. Dann gibt man 1,62 g 11 β-Fluor-7α-{5-[methyl(7,7,8,8,9,9,10,10,10-nonafluordecyl)amino]pentyl}-estra-1,3,5(10)-trien-3,17β-diol N-oxid sowie weitere 10 ml getrocknetes Dimethylsulfoxid zu und rührt 30 Minuten bei Raumtemperatur. Dann wird mit Essigester verdünnt, mit gesättigter Natriumhydrogencarbonatlösung, Wasser und Natriumchloridlösung gewaschen, über Natriumsulfat getrocknet, im Vakuum zur Trockne eingeengt und an Kieselgel mit Dichlormethan/Methanol chromatographiert. Man erhält 1,32 g 11β-Fluor-7α-{5-[methyl(7,7,8,8,9,9,10,10,10-nonafluordecyl)amino]pentyl}-estra-1,3,5(10)-trien-3-ol-17on N-oxid als Feststoff vom Schmelzpunkt 84-86°C; [α]_{D} = +53,6° in Chloroform.

### Verfahrensvariante 2.1:

(Herstellung von 17α-Methyl-estratrienen mit Amin-N-oxidgruppierung in der Seitenkette, ausgehend von 17-Oxo-estratrienen mit Amingruppierung in der Seitenkette über die entsprechenden 17-Oxo-estratriene mit Amin-N-oxidgruppierung in der Seitenkette):
**a) 11β-Fluor-7α-{5-[methyl(7,7,8,8,9,9,10,10,10-nonafluordecyl)amino]pentyl}-17α-methylestra-1,3,5(10)-trien-3,17β-diol** (physikalische Eigenschaften dieser Verbindung (Nr. 7) sind in Tabelle 1 angegeben)
   Eine Suspension von 230 g Cer-III-chlorid in 2,3 I Tetrahydrofuran wird bei 0°C mit 320 ml einer 3-molaren Methylmagnesiumbromidlösung in Diethylether versetzt und 30 Minuten gerührt. Dazu tropft man eine Lösung von 25 g 11β-Fluor-7α-{5-[methyl(7,7,8,8,9,9,10,10,10-nonafluordecyl)amino]pentyl}estra-1,3,5(10)-trien-3-ol-17-on (Drehwert α_{D} dieser Verbindung (Nr. Z14) ist in Tabelle 5 angegeben) in 250 ml Tetrahydrofuran und rührt anschließend 24 Stunden bei Raumtemperatur, versetzt bei 0°C mit Ammoniumchloridlösung, extrahiert mit Essigester, wäscht mit Wasser, trocknet mit Natriumsulfat, engt im Vakuum ein und chromatographiert an Kieselgel mit Dichlormethan/Methanol. Man erhält 19,1 g 11β-Fluor-7α-{5-[methyl(7,7,8,8,9,9,10,10,10-nonafluordecyl)amino]pentyl}-17α-methylestra-1,3,5(10)-trien-3,17β-diol vom Schmelzpunkt 82-85°C und [α]_{D} = +21,8° in Chloroform.
**b) 11β-Fluor-7α-{5-[methyl(7,7,8,8,9,9,10,10,10-nonafluordecyl)amino]pentyl}-17α-methylestra-1,3,5(10)-trien-3,17β-diol N-oxid**
   Eine Lösung von 18 g 11β-Fluor-7α-{5-[methyl(7,7,8,8,9,9,10,10,10-nonafluordecyl)amino]pentyl}-17α-methylestra-1,3,5(10)-trien-3,17β-diol in 180 ml Chloroform und 180 ml Methanol wird mit 2,57 g Natriumhydrogencarbonat und 16,2 ml einer 30 %igen Wasserstoffperoxidlösung versetzt und 48 Stunden bei Raumtemperatur gerührt. Dann wird mit Dichlormethan verdünnt, mit Wasser und Natriumthiosulfatlösung gewaschen, über Natriumsulfat getrocknet, im Vakuum zur Trockne eingeengt und mit Diethylether ausgerührt. Man erhält 18,4 g 11β-Fluor-7α-{5-[methyl(7,7,8,8,9,9,10,10,10-nonafluordecyl)amino]pentyl}-17α-methylestra-1,3,5(10)-trien-3,17β-diol N-oxid vom Schmelzpunkt 122°C.

### Verfahrensvariante 2.2:

(Herstellung von 17α-Methyl-estratrienen mit Amingruppierung in der Seitenkette, ausgehend von 17-Oxo-estratrienen über die entsprechenden 17α-Methyl-estratriene):
**a) 7α-(5-Brompentyl)-11β-fluor-17α-methylestra-1,3,5(10)-trien-3,17β-diol**
   Eine Suspension von 46,8 g Cer-III-chlorid in 0,47 I Tetrahydrofuran wird bei 0°C mit 63,8 ml einer 3-molaren Methylmagnesiumbromidlösung in Diethylether versetzt und 1 Stunde gerührt. Dazu tropft man eine Lösung von 25 g 7α-(5-Brompentyl)-11β-fluor-estra-1,3,5(10)-trien-3-ol-17-on in 200 ml Tetrahydrofuran und rührt anschließend 28 Stunden bei Raumtemperatur, versetzt bei 0°C mit Ammoniumchloridlösung, extrahiert mit Essigester, wäscht mit Wasser, trocknet mit Natriumsulfat, engt im Vakuum ein und chromatographiert an Kieselgel mit Dichlormethan/Methanol. Man erhält 15,1 g 7α-(5-Brompentyl)-11β-fluor-17α-methylestra-1,3,5(10)-trien-3,17β-diol vom Schmelzpunkt 48,6°C.
**b) 11β-Fluor-7α-{5-[methyl(7,7,8,8,9,9,10,10,10-nonafluordecyl)amino]pentyl}-17α-methylestra-1,3,5(10)-trien-3,17β-diol** (physikalische Eigenschaften dieser Verbindung (Nr. 7) sind in Tabelle 1 angegeben)
   Eine Lösung von 18 g 7α-(5-Brompentyl)-11β-fluor-17α-methylestra-1,3,5(10)-trien-3,17β-diol in 180 ml Dimethylformamid wird mit 15,9 g (7,7,8,8,9,9,10,10,10-Nonafluordecyl)-methyl-amin und 5 g Natriumcarbonat versetzt und anschließend 8,5 Stunden bei 80°C Badtemperatur gerührt. Dann wird auf Wasser gegeben, mit Essigester extrahiert, mit Wasser und gesättigter Natriumchloridlösung gewaschen, über Natriumsulfat getrocknet, im Vakuum eingeengt und chromatographiert an Kieselgel mit Dichlormethan/Methanol. Man erhält 22,9 g 11β-Fluor-7α-{5-[methyl(7,7,8,8,9,9,10,10,10-nonafluordecyl)amino]pentyl}-17α-methylestra-1,3,5(10)-trien-3,17β-diol vom Schmelzpunkt 82-85°C und [α]_{D} = +21,8° in Chloroform.

Weitere erfindungsgemässe Verbindungen können analog hergestellt werden. Hierzu sind zusätzlich Zwischenprodukte in Tabelle 5 aufgeführt. Ausserdem sind teilweise auch physikalische Eigenschaften dieser Verbindungen angegeben.

**Tabelle 1:**

| | | Schmelzpunkt [°C] | Drehwert α_{D} ¹) |
|---|---|---|---|
| 1 | 11 β-Fluor-7α-{5-[methyl(7,7,8,8,9,9,9-heptafluornonyl)amino]pentyl}-17α-methylestra-1,3,5(10)-trien-3,17β-diol N-oxid | 152-154 | |
| 2 | 11 β-Fluor-7α-{5-[methyl(8,8,9,9,10,10,10-heptafluordecyl)amino]pentyl}-17α-methylestra-1,3,5(10)-trien-3,17β-diol N-oxid | 137,7 | + 31° |
| 3 | (RS)-11β-Fluor-7α-{5-[methyl(7,7,8,8,9,9,10,10,10-nonafluordecyl)amino]pentyl}-17α-methylestra-1,3,5(10)-trien-3,17β-diol N-oxid | 122 | +29,6° |
| 4 | 11 β-Fluor-7α-{5-[methyl(8,8,9,9,9-pentafluornonyl)amino]pentyl}-17α-methylestra-1,3,5(10)-trien-3,17β-diol N-oxid | 148,5 | +25,3° |
| 5 | 11 β-Fluor-7α-{5-[methyl(9,9,10,10,10-pentafluordecyl)amino]pentyl-17α-methylestra-1,3,5(10)-trien-3,17β-diol N-oxid | 118-120 | + 26° |
| 6 | 11 β-Fluor-7α-{5-[methyl(8,8,9,9,9-pentafluornonyl)amino]pentyl}-17α-methylestra-1,3,5(10)-trien-3,17β-diol | 68-71 | + 32° |
| 7 | 11 β-Fluor-7α-{5-[methyl(7,7,8,8,9,9,10,10,10-nonafluordecyl)amino]pentyl}-17α-methylestra-1,3,5(10)-trien-3,17β-diol | 82-85 | + 21,8 |
| 8 | 11 β-Fluor-7α-{5-[methyl(7,7,8,8,9,9,9-heptafluornonyl)amino]pentyl}-17α-methylestra-1,3,5(10)-trien-3,17β-diol | 138 | + 29,8° |
| 9 | 17α-Ethinyl-11β-fluor-7α-{5-[methyl(7,7,8,8,9,9,10,10,10-nonafluordecyl)amino]pentyl}-estra-1,3,5(10)-trien-3,17β-diol | 128-130 | + 13,1° |
| 10 | 17α-Ethinyl-11β-fluor-3-(2-tetrahydropyranoyloxy)-7α-{5-[methyl(7,7,8,8,9,9,10,10,10-nonafluordecyl)amino]pentyl}-estra-1,3,5(10)-trien-17β-ol | | + 18,1° |
| 11 | 11 β-Fluor-3-(2-tetrahydropyranyloxy)-7α-{5-[methyl(7,7,8,8,9,9,10,10,10-nonafluordecyl)amino]pentyl}-17α-methylestra-1,3,5(10)-trien-17β-ol | | + 26,9° |
| 12 | 11 β-Fluor-7α-{5-[methyl(7,7,8,8,9,9,10,10,10-nonafluordecyl)amino]pentyl}-17α-trifluormethylestra-1,3,5(10)-trien-3,17β-diol | | + 24,6° |
| 13 | 11 β-Fluor-7α-{5-[methyl(6,6,7,7,8,8,8-heptafluoroctyl)amino]pentyl}-17α-methylestra-1,3,5(10)-trien-3,17β-diol | 96,3 | + 38,8° |
| 14 | 11 β-Fluor-7α-{5-[methyl(8,8,9,9,10,10,10-heptafluordecyl)amino]pentyl}-17α-methylestra-1,3,5(10)-trien-3,17β-diol | 137 | + 24,6° |
| 15 | 11 β-Fluor-7α-{5-[methyl(6,6,7,7,8,8,9,9,10,10,10-undecafluordecyl)amino]pentyl}-17α-methylestra-1,3,5(10)-trien-3,17β-diol | 112,6 | + 21,3° |
| 16 | 11 β-Fluor-7α-{5-[methyl(5,5,6,6,7,7,8,8,8-nonafluoroctyl)amino]pentyl}-17α-methylestra-1,3,5(10)-trien-3,17β-diol | | |
| 17 | 11 β-Fluor-7α-{5-[methyl(9,9,10,10,11,11,11-heptafluorundecyl)amino]pentyl}-17α-methylestra-1,3,5(10)-trien-3,17β-diol | | |
| 18 | 11 β-Fluor-7α-{5-[methyl(9,9,10,10,10-pentafluordecyl)amino]pentyl}-17α-methylestra-1,3,5(10)-trien-3,17β-diol | 88-90 | + 32,5° |

| | | | |
|---|---|---|---|
| ¹) [α]_{D} in Chloroform | | | |

**Tabelle 2:**

| | | Antiuterotrophe Wirkung | | | |
|---|---|---|---|---|---|
| | | s.c. | % Inhib. | p.o. | % Inhib. |
| 3 | (RS)-11β-Fluor-7α-{5-[methyl(7,7,8,8,9,9,10,10,10-nonafluordecyl)amino]pentyl}-17α-methylestra-1,3,5(10)-trien-3,17β-diol N-oxid | | | 0,3 | 76 |
| 7 | 11β-Fluor-7α-{5-[methyl(7,7,8,8,9,9,10,10,10-nonafluordecyl)amino]pentyl}-17α-methylestra-1,3,5(10)-trien-3,17β-diol | 0,03 | 59 | | |
| 8 | 11 β-Fluor-7α-{5-[methyl(7,7,8,8,9,9,9-heptafluornonyl)amino]pentyl}-17α-methylestra-1,3,5(10)-trien-3,17β-diol | | | 0,3 | 94 |

**Tabelle 3:**

| | | Schmelzpunkt [°C] | Drehwert α_{D} ¹) |
|---|---|---|---|
| X1 | 11β-Fluor-7α-{5-[methyl(8,8,9,9,9-pentafluornonyl)amino]pentyl}estra-1,3,5(10)-trien-3,17β-diol N-oxid | 158-160 | + 33,6° |
| X2 | 11 β-Fluor-7α-[5-(methyl{3-[(2,3,4,5,6-pentafluorphenyl)sulfanyl]propyl}amino)pentyl]estra-1,3,5(10)-trien-3,17β-diol N-oxid | | |
| X3 | 11 β-Fluor-7α-[5-(methyl{3-[(4,4,5,5,5-pentafluorpentyl)sulfanyl]propyl}amino)pentyl]estra-1,3,5(10)-trien-3,17β-diol N-oxid | 114-116 | |
| X4 | 11 β-Fluor-7α-[5-(methyl{3-[(4,4,5,5,5-pentafluorpentyl)sulfinyl]propyl}amino)pentyl]estra-1,3,5(10)-trien-3,17β-diol N-oxid | 103-105 | |
| X5 | 11 β-Fluor-7α-{5-[methyl(7,7,8,8,9,9,10,10,10-nonafluordecyl)amino]pentyl}estra-1,3,5(10)-trien-3,17β-diol N-oxid | 147-150 | + 30,2° |
| X6 | (S)-11β-Fluor-7α-{5-[methyl(7,7,8,8,9,9,10,10,10-nonafluordecyl)amino]pentyl}-17α-methylestra-1,3,5(10)-trien-3,17β-diol N-oxid | 128,5 | + 32,5° |
| X7 | (R)-11β-Fluor-7α-{5-[methyl(7,7,8,8,9,9,10,10,10-nonafluordecyl)amino]pentyl}-17α-methylestra-1,3,5(10)-trien-3,17β-diol N-oxid | 144,0 | + 31,3° |
| X8 | 11 β-Fluor-7α-{5-[methyl(9,9,10,10,10-pentyfluordecyl)amino]pentyl}estra-1,3,5(10)-trien-3,17β-diol N-oxid | 99-101 | + 28,5° |

| | | | |
|---|---|---|---|
| ¹) [α]_{D} in Chloroform | | | |

**Tabelle 4:**

| | | Schmelzpunkt [°C] | Drehwert α_{D} ¹) |
|---|---|---|---|
| Y1 | 11 β-Fluor-7α-[5-(methyl{3-[(4,4,5,5,5-pentafluorpentyl)sulfinyl]propyl}amino)pentyl]estra-1,3,5(10)-trien-3-ol-17-on N-oxid | | + 45,6° |
| Y2 | 11 β-Fluor-7α-[5-(methyl{3-[(4,4,5,5,5-pentafluorpentyl)sulfanyl]propyl}amino)pentyl]estra-1,3,5(10)-trien-3-ol-17-on N-oxid | | + 52,8° |
| Y3 | 11 β-Fluor-7α-{5-[methyl(7,7,8,8,9,9,10,10,10-nonafluordecyl)amino]pentyl}estra-1,3,5(10)-trien-3-ol-17-on N-oxid | 84-86 | + 53,6° |

| | | | |
|---|---|---|---|
| ¹) [α]_{D} in Chloroform | | | |

**Tabelle 5:**

| | | | |
|---|---|---|---|
| | | Schmelzpunkt [°C] | Drehwert α_{D} ¹) |
| Z1 | 11 β-Fluor-7α-{5-[methyl(8,8,9,9,9-pentafluornonyl)amino]pentyl}estra-1,3,5(10)-trien-3,17β-diol | | |
| Z2 | 11 β-Fluor-7α-{5-[methyl(8,8,9,9,9-pentafluornonyl)amino]pentyl}estra-1,3,5(10)-trien-3-ol-17-on | | |
| Z3 | 11β-Fluor-7α-{5-[methyl(3,3,4,4,5,5,6,6,7,7,8,8,8-tridecafluoroctyl)amino]pentyl}estra-1,3,5(10)-trien-3-ol-17-on | | |
| Z4 | 11 β-Fluor-7α-{5-[methyl(9,9,10,10,10-pentafluordecyl)amino]pentyl}estra-1,3,5(10)-trien-3-ol-17-on | | + 48,4° |
| Z5 | 11 β-Fluor-7α-{5-[methyl(9,9,10,10,10-pentafluordecyl)amino]pentyl}estra-1,3,5(10)-trien-3,17β-diol | | |
| Z6 | 11β-Fluor-7α-{5-[methyl(3,3,4,4,5,5,6,6,6-nonafluorhexyl)amino]pentyl}estra-1,3,5(10)-trien-3,17β-diol | | |
| Z7 | 11 β-Fluor-7α-{5-[methyl(4,4,5,5,6,6,7,7,8,8,9,9,9-tridecafluornonyl)amino]pentyl}estra-1,3,5(10)-trien-3,17β-diol | | |
| Z8 | 11 β-Fluor-7α-{5-[methyl(7,7,8,8,8-pentafluoroctyl)amino]pentyl}estra-1,3,5(10)-trien-3,17β-diol | | |
| Z9 | 11 β-Fluor-7α-{5-[methyl(7,7,8,8,9,9,10,10,10-nonafluordecyl)amino]pentyl}estra-1,3,5(10)-trien-3,17β-diol | | |
| Z10 | 11β-Fluor-7α-{5-[methyl(7,7,8,8,9,9,10,10,11,11,12,12,12-tridecafluordodecyl)amino]pentyl}estra-1,3,5(10)-trien-3,17β-diol | | |
| Z11 | 11β-Fluor-7α-{5-[methyl(4,4,5,5,6,6,7,7,8,8,9,9,10,10,11,11,11-heptadecafluorundecyl)amino]pentyl}estra-1,3,5(10)-trien-3,17β-diol | | |
| Z12 | 11β-Fluor-7α-{5-[methyl(7,7,8,8,9,9,10,10,11,11,12,12,13,13,14,14,14-heptadecafluortetradecyl)amino]pentyl}estra-1,3,5(10)-trien-3,17β-diol | | |
| Z13 | 11β-Fluor-7α-{5-[methyl(5,5,6,6,7,7,8,8,9,9,10,10,10-tridecafluordecyl)amino]pentyl}estra-1,3,5(10)-trien-3,17β-diol | | |
| Z14 | 11 β-Fluor-7α-{5-[methyl(7,7,8,8,9,9,10,10,10-nonafluordecyl)amino]pentyl}estra-1,3,5(10)-trien-3-ol-17-on | | + 58,2° |
| Z15 | 11 β-Fluor-3-methoxy-7α-{5-[methyl(7,7,8,8,9,9,10,10,10-nonafluordecyl)amino]pentyl}estra-1,3,5(10)-trien-17β-ol | | + 39,2° |
| Z16 | 11 β-Fluor-3-methoxy-7α-{5-[methyl(7,7,8,8,9,9,10,10,10-nonafluordecyl)amino]pentyl}estra-1,3,5(10)-trien-17-on | | + 55,9° |
| Z17 | 17β-Acetyloxy-11β-fluor-7α-{5-[methyl(7,7,8,8,9,9,10,10,10-nonafluordecyl)amino]pentyl}estra-1,3,5(10)-trien-3-ol | | + 21,0° |
| Z18 | 11 β-Fluor-7α-{5-[methyl(7,7,8,8,9,9,10,10,10-nonafluordecyl)amino]pentyl}estra-1,3,5(10)-trien-3-(2-tetrahydropyranoyloxy)-17-on | | + 66,1° |
| Z19 | 3-*tert*-Butanoyloxy-11β-fluor-7α-{5-[methyl(7,7,8,8,9,9,10,10,10-nonafluordecyl)amino]pentyl}estra-1,3,5(10)-trien-17β-ol | | + 31,2° |
| Z20 | 3-Acetyloxy-11β-fluor-7α-{5-[methyl(7,7,8,8,9,9,10,10,10-nonafluordecyl)amino]pentyl}estra-1,3,5(10)-trien-17β-ol | | + 33° |
| Z21 | 11 β-Fluor-7α-{5-[methyl(6,6,7,7,8,8,9,9,9-nonafluorunonyl)amino]pentyl}estra-1,3,5(10)-trien-3,17β-diol | | |
| Z22 | 11 β-Fluor-7α-{5-[methyl(8,8,9,9,10,10,11,11,11-nonafluorundecyl)amino]pentyl}estra-1,3,5(10)-trien-3,17β-diol | | |
| Z23 | 11 β-Fluor-7α-{5-[methyl(7,7,8,8,9,9,9-heptafluornonyl)amino]pentyl}estra-1,3,5(10)-trien-3,17β-diol | 125,0 | |
| Z24 | 11 β-Fluor-7α-{5-[methyl(7,7,8,8,9,9,9-heptafluornonyl)amino]pentyl}estra-1,3,5(10)-trien-3-ol-17-on | | + 70,2° |
| Z25 | 11 β-Fluor-7α-{5-[methyl(6,6,7,7,8,8,8-heptafluoroctyl)amino]pentyl}estra-1,3,5(10)-trien-3-ol-17-on | | + 71,6° |
| Z26 | 11 β-Fluor-7α-{5-[methyl(6,6,7,7,8,8,8-heptafluoroctyl)amino]pentyl}estra-1,3,5(10)-trien-3,17β-diol | 112,8 | + 42,6° |
| Z27 | 11 β-Fluor-7α-{5-[methyl(8,8,9,9,10,10,10-heptafluordecyl)amino]pentyl}estra-1,3,5(10)-trien-3-ol-17-on | | + 56,2° |
| Z28 | 11 β-Fluor-7α-{5-[methyl(8,8,9,9,10,10,10-heptafluordecyl)amino]pentyl}estra-1,3,5(10)-trien-3,17β-diol | 104 | + 34,9° |
| Z29 | 11 β-Fluor-7α-{5-[methyl(6,6,7,7,8,8,9,9,10,10,10-undecafluordecyl)amino]pentyl}estra-1,3,5(10)-trien-3-ol-17-on | | + 64,6° |
| Z30 | 11 β-Fluor-7α-{5-[methyl(6,6,7,7,8,8,9,9,10,10,10-undecafluordecyl)amino]pentyl}estra-1,3,5(10)-trien-3,17β-diol | 94-96 | + 36,8° |
| Z31 | 11 β-Fluor-7α-{5-[methyl(5,5,6,6,7,7,8,8,8-nonafluoroctyl)amino]pentyl}estra-1,3,5(10)-trien-3-ol-17-on | | |
| Z32 | 11 β-Fluor-7α-{5-[methyl(5,5,6,6,7,7,8,8,8-nonafluoroctyl)amino]pentyl}estra-1,3,5(10)-trien-3,17β-diol | | |
| Z33 | 11 β-Fluor-7α-{5-[methyl(9,9,10,10,11,11,11-heptafluorundecyl)amino]pentyl}estra-1,3,5(10)-trien-3-ol-17-on | | |
| Z34 | 11 β-Fluor-7α-{5-[methyl(9,9,10,10,11,11,11-heptafluorundecyl)amino]pentyl}estra-1,3,5(10)-trien-3,17β-diol | | |

| | | | |
|---|---|---|---|
| ¹) [α_{D}] in Chloroform | | | |

## Patentansprüche

1. 17α-Alkyl-17β-oxy-estratriene mit der allgemeinen Formel I 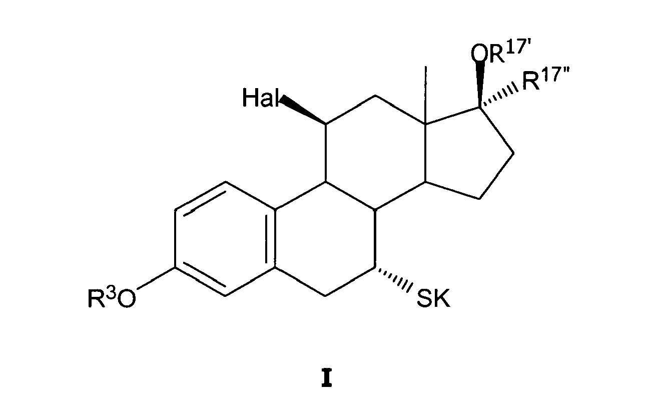 worin
**Hal** für F oder CI steht und in 11 β-Position an das Estratriengrundgerüst gebunden ist,
**R³** für Wasserstoff, C₁- - C₄-Alkyl, C₁- - C₄-Alkanoyl oder einen cyclischen C₃- - C₇-Ether mit einem O-Atom steht,
**R^{17'}** für Wasserstoff, C₁- - C₄-Alkyl oder C₁- - C₄-Alkanoyl steht,
**R^{17"}** für C₁- - C₄-Alkyl, C₁- - C₄-Alkyl, C₁- - C₄-Alkinyl sowie für zumindest teilweise fluorierte C₁- - C₄-Alkylreste, steht,
wobei **R^{17'}**-O in 17β-Position und **R^{17"}** in 17α-Position an das Estratriengrundgerüst gebunden ist, und
**SK** für die Gruppierung **U-V-W-X-Y-Z-E** steht, wobei diese Gruppierung über **U** in 7α-Position an das Estratriengerüst gebunden ist,
worin **U** entweder einen gerad- oder verzweigtkettigen C₁- - C₁₃-Alkylen-, -Alkenylen- oder -Alkinylenrest oder die Gruppe **A-B** darstellt, wobei **A** an das Estratriengrundgerüst gebunden ist und einen über -CH₂- an das Estratriengrundgerüst gebundenen Benzylidenrest, einen Phenylenrest oder einen über die Alkylgruppe an das Estratriengrundgerüst gebundenen C₁- - C₃-Alkylarylrest darstellt und **B** für einen gerad- oder verzweigtkettigen C₁- - C₁₃-Alkylen-, -Alkenylen- oder -Alkinylenrest steht und wobei **A** und **B** auch über ein O-Atom miteinander verbunden sein können,
worin weiter **V** eine CH₂- oder eine C(O)-Gruppe darstellt, worin ferner **W** eine N⁺(O⁻)(**R⁶**)-Gruppe oder ein Azolidinylen-N-Oxid-Ring ist, wobei der Azolidinylen-N-Oxid-Ring mindestens ein C-Atom der Gruppierung X einschliesst,
wobei ferner **R⁶** entweder H oder CH₂-**R⁷** oder C(O)-**R⁷** ist, worin **R⁷** folgendes bedeuten kann:
a) Wasserstoff oder
b) einen gerad- oder verzweigtkettigen, nichtfluorierten oder zumindest teilweise fluorierten C₁- - C₁₄-Alkyl-, -Alkenyl- oder -Alkinylrest, der einfach oder mehrfach hydroxyliert sein kann und durch ein bis drei der Heteroatome -O- und -S- und/oder der Gruppierungen -**NR⁹**- unterbrochen sein kann, worin **R⁹** für Wasserstoff oder einen C₁- - C₃-Alkylrest steht, oder
c) einen unsubstituierten oder substituierten Aryl- oder Heteroarylrest oder
d) einen unsubstituierten oder substituierten C₃- - C₁₀-Cycloalkylrest oder
e) einen unsubstituierten oder substituierten C₄- - C₁₅-Cycloalkylalkylrest oder
f) einen unsubstituierten oder substituierten C₇- - C₂₀-Aralkylrest oder
g) einen unsubstituierten oder substituierten Heteroaryl-C₁- - -C₆-alkylrest oder
h) einen unsubstituierten oder substituierten Aminoalkylrest oder einen Biphenylrest,
worin weiter **X** ein gerad- oder verzweigtkettiger C₁- - C₁₂-Alkylen-, -Alkenylen- oder -Alkinylenrest ist,
worin weiter Y eine direkte Bindung zwischen **X** und **Z** ist oder folgendes bedeuten kann:
a) eine SOₙ-**R¹⁰**-Gruppe, wobei n = 0,1 oder 2 ist und wobei **R¹⁰** eine direkte Bindung zwischen SOₙ und **Z** oder einen gerad- oder verzweigtkettigen C₁- - C₆-Alkylen-, -Alkenylen- oder -Alkinylenrest darstellt oder
b) die Gruppe **R¹¹** oder O-**R¹¹**, wobei **R¹¹** für
i) einen gerad- oder verzweigtkettigen C₁- - C₅-Alkylen-, -Alkenylen- oder -Alkinylenrest steht oder für
ii) einen unsubstituierten oder substituierten Arylrest oder Heteroarylrest steht oder für
iii) einen unsubstituierten oder substituierten C₃- - C₁₀-Cyclalkylrest steht oder für
iv) einen unsubstituierten oder substituierten C₄- - C₁₅-Cycloalkylalkylrest steht oder für
v) einen unsubstituierten oder substituierten C₇- - C₂₀-Aralkylrest steht oder für
vi) einen unsubstituierten oder substituierten Heteroaryl-C₁- - -C₆-alkylrest steht, oder
c) die Gruppierung CH = CF oder
d) die Gruppierung HN-C(O)-NH-**R¹²**, wobei **R¹²** für einen unsubstituierten oder substituierten Arylenrest steht und wobei **R¹²** an **Z** gebunden ist, und
worin weiter Z eine direkte Bindung zwischen Y und E oder ein gerad- oder verzweigtkettiger C₁- - C₉-Alkylen-, -Alkenylen- oder -Alkinylenrest ist, der teilweise oder vollständig fluoriert sein kann, und
worin weiter **E** eine CF₃-Gruppe oder eine zumindest teilweise fluorierte Arylgruppe ist,
wobei auch pharmakologisch verträgliche Säureadditionssalze sowie Ester umfasst sind.

2. Estratriene nach Anspruch 1, **dadurch gekennzeichnet, dass R³** für Wasserstoff, CH₃, CH₃CO oder C₅H₁₀O steht.

3. Estratriene nach einem der vorstehenden Ansprüche, **dadurch gekennzeichnet, dass R^{17'}** für Wasserstoff, CH₃ oder CH₃CO steht und dass **R^{17"}** für Methyl, Ethinyl oder Trifluormethyl steht.

4. Estratriene nach einem der vorstehenden Ansprüche, **dadurch gekennzeichnet, dass Hal** für Fluor steht.

5. Estratriene nach einem der vorstehenden Ansprüche, **dadurch gekennzeichnet, dass U** für (CH₂)ₚ steht, wobei p eine ganze Zahl von 2 - 10 ist.

6. Estratriene nach einem der vorstehenden Ansprüche, **dadurch gekennzeichnet, dass** p = 4 ist.

7. Estratriene nach einem der vorstehenden Ansprüche, **dadurch gekennzeichnet, dass V** für CH₂ steht.

8. Estratriene nach einem der vorstehenden Ansprüche, **dadurch gekennzeichnet, dass W** für N⁺(O⁻)(**R⁶**) steht, wobei **R⁶** Wasserstoff oder ein C₁- - C₃-Alkylrest ist.

9. Estratriene nach einem der vorstehenden Ansprüche, **dadurch gekennzeichnet, dass R⁶** für Methyl steht.

10. Estratriene nach einem der vorstehenden Ansprüche, **dadurch gekennzeichnet, dass X** für (CH₂)_{q} steht, wobei q = 0 oder eine ganze Zahl von 1 - 12 ist.

11. Estratriene nach einem der vorstehenden Ansprüche, **dadurch gekennzeichnet, dass Y** eine direkte Bindung zwischen X und Z.

12. Estratriene nach einem der vorstehenden Ansprüchen, **dadurch gekennzeichnet, dass Z** ein gerad- oder verzweigtkettiger C₁- - C₇-Alkylenrest ist, der zumindest teilweise fluoriert ist.

13. Estratriene nach einem der vorstehenden Ansprüche, **dadurch gekennzeichnet, dass E** für CF₃ oder für Pentafluorphenyl steht.

14. Estratriene nach einem der vorstehenden Ansprüchen, **dadurch gekennzeichnet, dass Z-E** für C₂F₅, C₃F₇, C₄F₉ oder für C₆F₅ steht.

15. Estratriene mit der allgemeinen Formel I nach Anspruch 1, nämlich
11 β-Fluor-7α-{5-[methyl(7,7,8,8,9,9,9-heptafluornonyl)amino]pentyl}-17α-methylestra-1,3,5(10)-trien-3,17β-diol N-oxid
11 β-Fluor-7α-{5-[methyl(8,8,9,9,10,10,10-heptafluordecyl)amino]pentyl}-17α-methylestra-1,3,5(10)-trien-3,17β-diol N-oxid
(RS)-11β-Fluor-7α-{5-[methyl(7,7,8,8,9,9,10,10,10-nonafluordecyl)amino]pentyl}-17α-methylestra-1,3,5(10)-trien-3,17β-diol N-oxid
11 β-Fluor-7α-{5-[methyl(8,8,9,9,9-pentafluornonyl)amino]pentyl}-17α-methylestra-1,3,5(10)-trien-3,17β-diol N-oxid
11 β-Fluor-7α-{5-[methyl(9,9,10,10,10-pentafluordecyl)amino]pentyl-17α-methylestra-1,3,5(10)-trien-3,17β-diol N-oxid

16. Verwendung der 17α-Alkyl-17β-oxy-estratriene mit der allgemeinen Formel I nach einem der Ansprüche 1 - 15 zur Herstellung von Arzneimitteln.

17. Pharmazeutische Präparate, enthaltend mindestens ein 17α-Alkyl-17β-oxy-estratrien mit der allgemeinen Formel I nach einem der Ansprüche 1 - 15 sowie mindestens einen pharmazeutisch verträglichen Träger.

18. 17β-Oxy-estratriene mit der allgemeinen Formel II 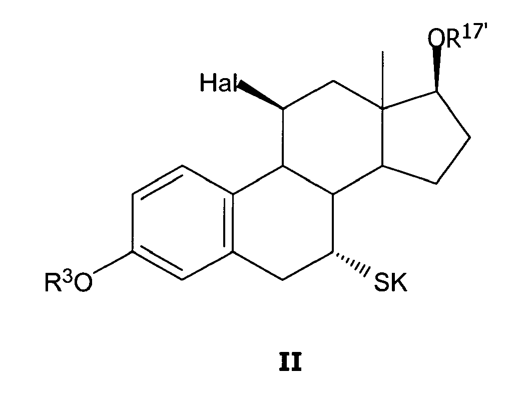 worin
**Hal** für F oder CI steht und in 11 β-Position an das Estratriengrundgerüst gebunden ist,
**R³** für Wasserstoff, C₁- - C₄-Alkyl, C₁- - C₄-Alkanoyl oder einen cyclischen C₃- - C₇-Ether mit einem O-Atom steht,
**R^{17'}** für Wasserstoff, C₁- - C₄-Alkyl oder für C₁- - C₄-Alkanoyl steht und in 17β-Position an das Estratriengrundgerüst gebunden ist, und
**SK** für die Gruppierung **U-V-W-X-Y-Z-E** steht, wobei diese Gruppierung über **U** in 7α-Position an das Estratriengerüst gebunden ist und wobei **U, V, X, Y, Z** und **E** die in den Ansprüchen 1 - 15 angegebenen Bedeutungen haben, mit der Massgabe, dass **W** für eine N⁺(O⁻)(**R⁶**)-Gruppe oder für einen Azolidinylen-N-Oxid-Ring steht, wobei der Azolidinylen-N-Oxid-Ring mindestens ein C-Atom der Gruppierung X einschliesst, worin **R⁶** die in einem der Ansprüche 1 - 15 angegebene Bedeutung hat.

19. 17β-Oxy-estratriene mit der allgemeinen Formel II, nämlich
11β-Fluor-7α-{5-[methyl(8,8,9,9,9-pentafluornonyl)amino]pentyl}estra-1,3,5(10)-trien-3,17β-diol N-oxid
11 β-Fluor-7α-[5-(methyl{3-[(2,3,4,5,6-pentafluorphenyl)sulfanyl]propyl}amino)pentyl]estra-1,3,5(10)-trien-3,17β-diol N-oxid
11 β-Fluor-7α-[5-(methyl{3-[(4,4,5,5,5-pentafluorpentyl)sulfanyl]propyl}amino)pentyl]estra-1,3,5(10)-trien-3,17β-diol N-oxid
11 β-Fluor-7α-[5-(methyl{3-[(4,4,5,5,5-pentafluorpentyl)sulfinyl]propyl}amino)pentyl]estra-1,3,5(10)-trien-3,17β-diol N-oxid
11 β-Fluor-7α-{5-[methyl(7,7,8,8,9,9,10,10,10-nonafluordecyl)amino]pentyl}estra-1,3,5(10)-trien-3,17β-diol N-oxid
(S)-11 β-Fluor-7α-{5-[methyl(7,7,8,8,9,9,10,10,10-nonafluordecyl)amino]pentyl}-17α-methylestra-1,3,5(10)-trien-3,17β-diol N-oxid
(R)-11 β-Fluor-7α-{5-[methyl(7,7,8,8,9,9,10,10,10-nonafluordecyl)amino]pentyl}-17α-methylestra-1,3,5(10)-trien-3,17β-diol N-oxid
11 β-Fluor-7α-{5-[methyl(9,9,10,10,10-pentyfluordecyl)amino]pentyl}estra-1,3,5(10)-trien-3,17β-diol N-oxid.

20. 17-Oxo-estratriene mit der allgemeinen Formel III 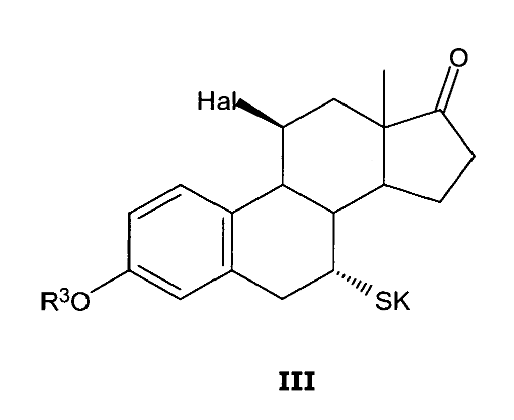 worin
**Hal für F** oder Cl steht und in 11 β-Position an das Estratriengrundgerüst gebunden ist,
**R³** für Wasserstoff, C₁- - C₄-Alkyl, C₁- - C₄-Alkanoyl oder einen cyclischen C₃- - C₇-Ether mit einem O-Atom steht, und
**SK** für die Gruppierung **U-V-W-X-Y-Z-E** steht, wobei diese Gruppierung über **U** in 7α-Position an das Estratriengerüst gebunden ist und wobei **U, V, X, Y, Z** und E die in den Ansprüchen 1 - 15 angegebenen Bedeutungen haben, mit der Massgabe, dass **W** für eine N⁺(O⁻)(**R⁶**)-Gruppe oder für einen Azolidinylen-N-Oxid-Ring steht, wobei der Azolidinylen-N-Oxid-Ring mindestens ein C-Atom der Gruppierung X einschliesst, worin **R⁶** die in einem der Ansprüche 1 - 15 angegebene Bedeutung hat.

21. 17-Oxo-estratriene mit der allgemeinen Formel III, nämlich
11 β-Fluor-7α-[5-(methyl{3-[(4,4,5,5,5-pentafluorpentyl)sulfinyl]propyl}amino)pentyl]estra-1,3,5(10)-trien-3-ol-17-on N-oxid
11 β-Fluor-7α-[5-(methyl{3-[(4,4,5,5,5-pentafluorpentyl)sulfanyl]propyl}amino)pentyl]estra-1,3,5(10)-trien-3-ol-17-on N-oxid
11 β-Fluor-7α-{5-[methyl(7,7,8,8,9,9,10,10,10-nonafluordecyl)amino]pentyl}estra-1,3,5(10)-trien-3-ol-17-on N-oxid
